# EUROPEAN PATENT APPLICATION

(11) **EP 4 806 346 A1**
(43) Date of publication of application: **16.09.2026**
(21) Application number: 26162881.2
(22) Date of filing: 06.03.2026
(51) Int. Cl.: A61B 5/11, A61B 5/395, A61B 5/00

(54) **MUSCLE RELAXATION MONITORING DEVICE AND MEDICAL DEVICE SYSTEM**

(30) Priority: 14.03.2025 JP 2025041822
(71) Applicant: Nihon Kohden Corporation, Shinjuku-ku Tokyo (JP); Takagi, Shunichi, Tokyo (JP)
(72) Inventor: Yoshihara, Hiroshi, Tokyo (JP); Onodera, Hiroshi, Tokorozawa-shi, Saitama (JP); Takagi, Shunichi, Tokorozawa-shi, Saitama (JP)
(74) Representative: Grünecker Patent- und Rechtsanwälte PartG mbB

(57) **Abstract**

A muscle relaxation monitoring device includes: a stimulator configured to apply a stimulation having a predetermined current value and a predetermined pulse width value to a nerve of a living body; and a physiological response detector configured to detect a physiological response. The stimulator applies a first stimulation having a first current value and a first pulse width value, and applies a second stimulation having a second current value and a second pulse width value after applying the first stimulation. The stimulator compares the physiological response detected when the first stimulation is applied with the physiological response detected when the second stimulation is applied, and adjusts the predetermined current value and/or the predetermined pulse width value to be applied to the living body.

## Description

### TECHNICAL FIELD

The presently disclosed subject matter relates to a muscle relaxation monitoring device and a medical device system.

### BACKGROUND ART

In the related art, a muscle relaxation monitoring device that applies a stimulation current to a patient is known (for example, JP2021-069723A). Such a muscle relaxation monitoring device stimulates a nerve of the patient by applying the stimulation current to the patient to move the muscle. Then, a potential is generated when the muscle of the patient is moved, and it is determined that a muscle relaxant is not effective as the potential increases. On the other hand, the smaller the potential is, the more effective the muscle relaxant is.

Here, an optimum value of the stimulation current applied to the patient varies depending on the patient. Therefore, it is necessary to perform calibration so that the value of the stimulation current becomes an optimum value for the patient. This calibration is performed before the muscle relaxant is administered, in other words, before surgery, because a correct amplitude cannot be obtained after administration of the muscle relaxant to the patient.

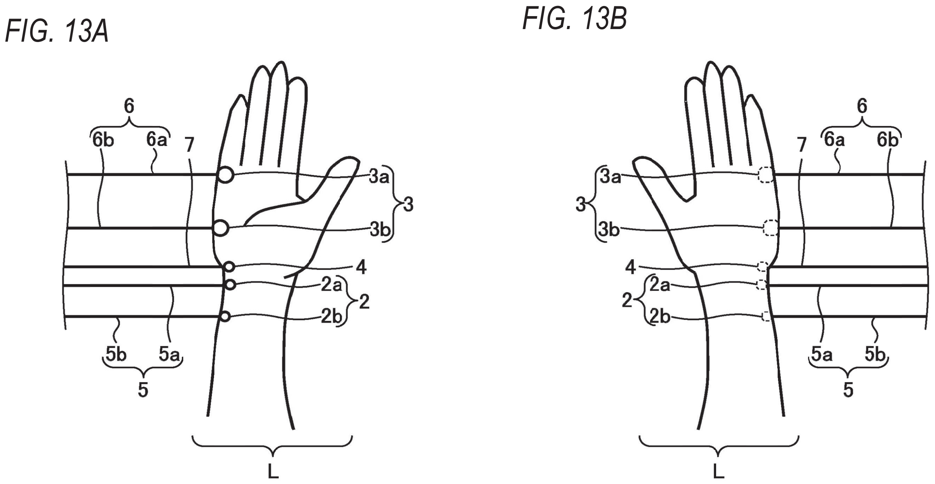

From the recent academic presentations and research papers, there is a possibility that a stimulation current required during surgery is insufficient due to a change in a body position of the patient or a change in a skin condition of the patient. For example, as illustrated in FIG. 13A and FIG. 13B, when a hand of the patient rotates from a position illustrated in FIG. 13A to a position illustrated in FIG. 13B, the nerve of the patient moves accordingly and an amplitude decreases, and therefore a value of the required stimulation current changes.

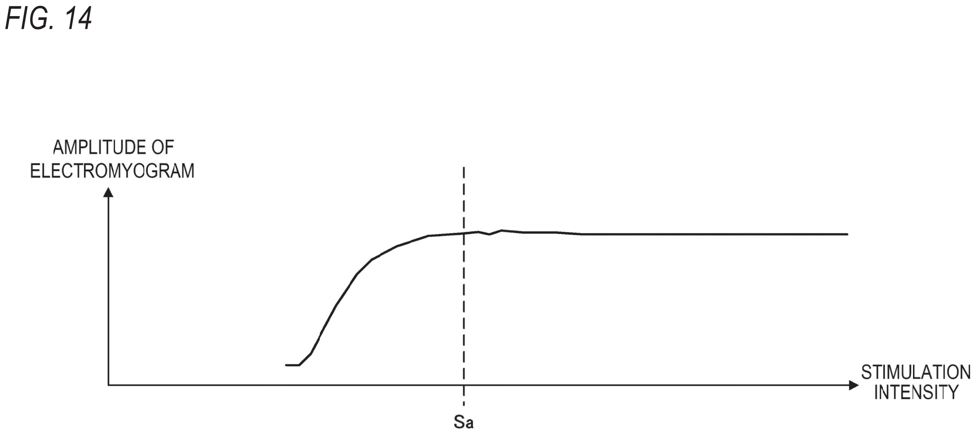

On the other hand, as illustrated in FIG. 14, when the value of the stimulation current applied to the patient is increased, the amplitude saturates at a certain timing (specifically, a timing Sa in FIG. 14), and thereafter, the amplitude becomes constant even when the value of the stimulation current is increased. Therefore, it is conceivable to set the value of the stimulation current to a large value in advance, in order to prevent insufficient stimulation current. However, when the value of the stimulation current is set to the large value in advance, distortion occurs in a waveform of an electromyogram due to a stimulation artifact noise, and a correct amplitude cannot be obtained. For these reasons, in the surgery performed for a long time, there is a limit in setting the value of the stimulation current to an optimum value during the surgery only by performing the calibration before the surgery.

### SUMMARY

In view of the above problems, an object of the presently disclosed subject matter is to provide a muscle relaxation monitoring device and a medical device system capable of setting a value of a stimulation current to an optimum value during surgery.

In order to solve such a problem, a muscle relaxation monitoring device according to the presently disclosed subject matter includes a stimulator configured to apply a stimulation having a predetermined current value and a predetermined pulse width value to a nerve of a living body, and a physiological response detector configured to detect a physiological response, in which the stimulator applies a first stimulation having a first current value and a first pulse width value, and applies a second stimulation having a second current value and a second pulse width value after applying the first stimulation, and compares the physiological response detected when the first stimulation is applied with the physiological response detected when the second stimulation is applied, and adjusts the predetermined current value and/or the predetermined pulse width value to be applied to the living body.

The muscle relaxation monitoring device according to the presently disclosed subject matter further includes a time measurer configured to perform predetermined time measurement, in which the time measurer starts measuring a preparation time based on satisfaction of a predetermined condition, and the stimulator applies the first stimulation based on measurement of a first time shorter than the preparation time with reference to a stimulation applied immediately before the preparation time is measured or a stimulation applied immediately after the preparation time is measured, when the preparation time is measured by the time measurer.

A medical device system according to the presently disclosed subject matter includes a muscle relaxation monitoring device configured to monitor a muscle relaxation degree of a patient, and a medical device configured to communicate with the muscle relaxation monitoring device, in which the muscle relaxation monitoring device includes a stimulator configured to apply a stimulation having a predetermined current value and a predetermined pulse width value to a nerve of a living body, a physiological response detector configured to detect a physiological response, and a communication unit configured to output information to the medical device, the stimulator applies a first stimulation having a first current value and a first pulse width value, and applies a second stimulation having a second current value and a second pulse width value after applying the first stimulation, and compares the physiological response detected when the first stimulation is applied with the physiological response detected when the second stimulation is applied, and adjusts the predetermined current value and the predetermined pulse width value to be applied to the living body, the communication unit outputs comparison information on a result of the comparison to the medical device, and the medical device includes a medical device controller that performs a control according to the comparison information output from the communication unit.

According to the presently disclosed subject matter, it is possible to provide a muscle relaxation monitoring device and a medical device system capable of setting a value of a stimulation current to an optimum value during surgery.

### BRIEF DESCRIPTION OF DRAWINGS

FIG. 1 illustrates a configuration of a medical device system.
FIG. 2 illustrates myoelectric-derived relaxation degrees.
FIG. 3 is a flowchart of a process performed by a muscle relaxation monitoring device.
FIG. 4 illustrates a subroutine of an intraoperative calibration process.
FIG. 5 illustrates a relationship between a value of a stimulation current to be added and a waiting time.
FIG. 6 illustrates an example of a stimulation intensity.
FIG. 7 is a timing chart of intraoperative calibration.
FIG. 8 is a timing chart when a TOF ratio is detected.
FIG. 9 illustrates a subroutine of an intraoperative calibration process in a second embodiment.
FIG. 10 is a timing chart of intraoperative calibration in the second embodiment.
FIG. 11 illustrates a subroutine of an intraoperative calibration process in a third embodiment.
FIG. 12 is a timing chart of intraoperative calibration in the third embodiment.
FIG. 13A is a diagram when an arm of a patient is rotated in a state in which an electrode is attached.
FIG. 13B is a diagram when an arm of a patient is rotated in a state in which an electrode is attached.
FIG. 14 illustrates a relationship among an amplitude of an electromyogram, a stimulation intensity, and a saturation point.

### DESCRIPTION OF EMBODIMENTS

### First Embodiment

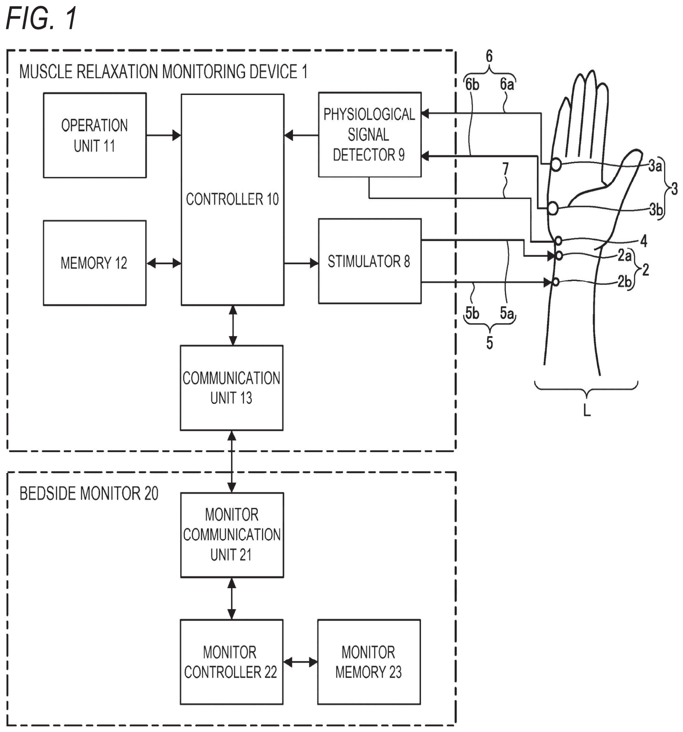

Hereinafter, a first embodiment according to the presently disclosed subject matter will be described with reference to FIGS. 1 to 8.

### Medical Device System in First Embodiment

First, a medical device system according to the first embodiment of the presently disclosed subject matter will be described with reference to FIG. 1.

The medical device system according to the first embodiment can include a muscle relaxation monitoring device 1, a stimulation electrode 2, a recording electrode 3, a neutral electrode 4, a wiring 5, a signal line 6, a ground line 7, and a bedside monitor 20.

The muscle relaxation monitoring device 1 is a device for monitoring a muscle relaxation degree of a patient who is a living body to which a muscle relaxant is administered. The muscle relaxation monitoring device 1 can include a stimulator 8, a physiological signal detector 9, a controller 10, an operation unit 11, a memory 12, and a communication unit 13.

The stimulation electrode 2 can include a first stimulation electrode 2a and a second stimulation electrode 2b, and is attached to a position corresponding to an ulnar nerve of an upper limb L of the patient. The stimulation electrode 2 is connected to the stimulator 8 via the wiring 5. Specifically, the first stimulation electrode 2a is connected to the stimulator 8 via a first wiring 5a. On the other hand, the second stimulation electrode 2b is connected to the stimulator 8 via a second wiring 5b.

The recording electrode 3 can include a first recording electrode 3a and a second recording electrode 3b, and is attached to positions corresponding to the abductor digiti minimi muscle of the upper limb L of the patient and a tendon in the vicinity thereof. The recording electrode 3 is connected to the physiological signal detector 9 via the signal line 6. Specifically, the first recording electrode 3a is connected to the physiological signal detector 9 via a first signal line 6a. On the other hand, the second recording electrode 3b is connected to the physiological signal detector 9 via a second signal line 6b.

The neutral electrode 4 is provided to reduce an influence of a noise voltage transmitted from the body of the patient through the upper limb L on a physiological response (specifically, a physiological signal) detected through the recording electrode 3. The neutral electrode 4 is connected to the physiological signal detector 9 via the ground line 7. The noise voltage is caused by propagation of a voltage pulse caused by the stimulation current in the body of the patient.

The wiring 5 can include the first wiring 5a and the second wiring 5b, and is provided to connect the stimulation electrode 2 and the stimulator 8. Specifically, the first wiring 5a is provided to connect the first stimulation electrode 2a and the stimulator 8. On the other hand, the second wiring 5b is provided to connect the second stimulation electrode 2b and the stimulator 8.

The signal line 6 can include the first signal line 6a and the second signal line 6b, and is provided to connect the recording electrode 3 and the physiological signal detector 9. Specifically, the first signal line 6a is provided to connect the first recording electrode 3a and the physiological signal detector 9. On the other hand, the second signal line 6b is provided to connect the second recording electrode 3b and the physiological signal detector 9.

The ground line 7 is provided to connect the neutral electrode 4 and the physiological signal detector 9. The stimulator 8 is provided to stimulate the ulnar nerve of the upper limb L of the patient by applying a pulsed stimulation current through the wiring 5 and the stimulation electrode 2. The physiological signal detector 9 detects a potential difference between two physiological signals generated in the recording electrode 3 and the abductor digiti minimi muscle and the tendon of the upper limb L of the patient input via the signal line 6. The stimulator 8 constitutes a "stimulator" in the presently disclosed subject matter, and the physiological signal detector 9 constitutes a "physiological response detector" in the presently disclosed subject matter.

The controller 10 can include a CPU and the like, and controls the stimulator 8 and the communication unit 13 by reading an operation program stored in the memory 12 and performing a predetermined calculation process. In addition, the controller 10 performs a process of storing information on the physiological signal input from the physiological signal detector 9 in the memory 12. In addition, the controller 10 controls the muscle relaxation monitoring device 1 when a signal output from the operation unit 11 is input. In addition, the controller 10 calculates an amplitude of the physiological signal based on an intensity change of the physiological signal detected by the physiological signal detector 9, and calculates the myoelectric-derived relaxation degree using the amplitude.

The operation unit 11 is provided to input a command from a user of the muscle relaxation monitoring device 1. The operation unit 11 may be a button, a touch panel, a keyboard, a mouse, a trackball, or the like.

The memory 12 is provided to store an operation program of the muscle relaxation monitoring device 1, a result of the predetermined calculation process performed by the controller 10, and the like. Specifically, the memory 12 is provided to store the myoelectric-derived relaxation degree calculated by the controller 10. The memory 12 can include a waiting time timer TMa for measuring a waiting time, a first standby time timer TMb for measuring a first standby time, and a second standby time timer TMc for measuring a second standby time. The memory 12 may be a recording medium such as a hard disk, a flexible disk, an MO, an MT, a RAM, a CD-ROM, a DVD-ROM, an SD card, a CF card, or a USB memory, or a server.

The communication unit 13 is provided to output a predetermined calculation result by the controller 10 to a monitor communication unit 21. Specifically, the communication unit 13 is provided to output a monitoring result of the muscle relaxation degree by the muscle relaxation monitoring device 1 and comparison information to the monitor communication unit 21. In addition, the communication unit 13 is provided to output, to the monitor communication unit 21, information on the myoelectric-derived relaxation degree calculated by the controller 10. The communication unit 13 is provided to input predetermined information from the monitor communication unit 21. The communication unit 13 constitutes a "communication unit" in the presently disclosed subject matter.

The "comparison information" can include information on a stimulation intensity having a value of a stimulation current and a value of a stimulation pulse width in an intraoperative calibration stimulation process (first time) to be described later, information on a stimulation intensity having a value of a stimulation current and a value of a stimulation pulse width in an intraoperative calibration stimulation process (second time) to be described later, information on a stimulation intensity having a value of a stimulation current and a value of a stimulation pulse width at the time of addition to be described later, and the like. The stimulation intensity can be calculated by the product of the value of the stimulation current and the value of the stimulation pulse width.

The bedside monitor 20 is provided to display a predetermined calculation result output from the communication unit 13. Specifically, the bedside monitor 20 is provided to display the monitoring result of the muscle relaxation degree by the muscle relaxation monitoring device 1 and the comparison information. The bedside monitor 20 can include the monitor communication unit 21, a monitor controller 22, and a monitor memory 23. The bedside monitor 20 is an example of a "medical device" in the presently disclosed subject matter.

The monitor communication unit 21 is provided to input the predetermined calculation result output from the communication unit 13. The monitor communication unit 21 is provided to output a calculation result by the monitor controller 22 to the communication unit 13.

The monitor controller 22 can include a CPU and the like, and controls the bedside monitor 20 by reading an operation program stored in the monitor memory 23 and performing a predetermined calculation process. The monitor memory 23 is provided to store the operation program of the bedside monitor 20, a result of the predetermined calculation process performed by the monitor controller 22, and the like. The monitor controller 22 constitutes a "medical device controller" in the presently disclosed subject matter.

The monitor memory 23 may be a recording medium such as a hard disk, a flexible disk, an MO, an MT, a RAM, a CD-ROM, a DVD-ROM, an SD card, a CF card, or a USB memory, or a server.

Here, a method of using the muscle relaxation monitoring device 1 will be described. First, the stimulation electrode 2, the recording electrode 3, and the neutral electrode 4 are attached to the upper limb L of the patient. Next, the stimulator 8 causes a stimulation current to flow between the first stimulation electrode 2a and the second stimulation electrode 2b via the first wiring 5a and the second wiring 5b to stimulate the ulnar nerve of the upper limb L of the patient. Next, the physiological signal detector 9 detects the potential difference between the two physiological signals generated in the abductor digiti minimi muscle and the tendon of the upper limb L of the patient based on the flow of the stimulation current by the stimulator 8. Then, based on the potential difference of the physiological signal detected by the physiological signal detector 9, a physiological waveform (electromyogram waveform) is obtained to monitor the muscle relaxation degree of the patient.

### Myoelectric-Derived Relaxation Degree

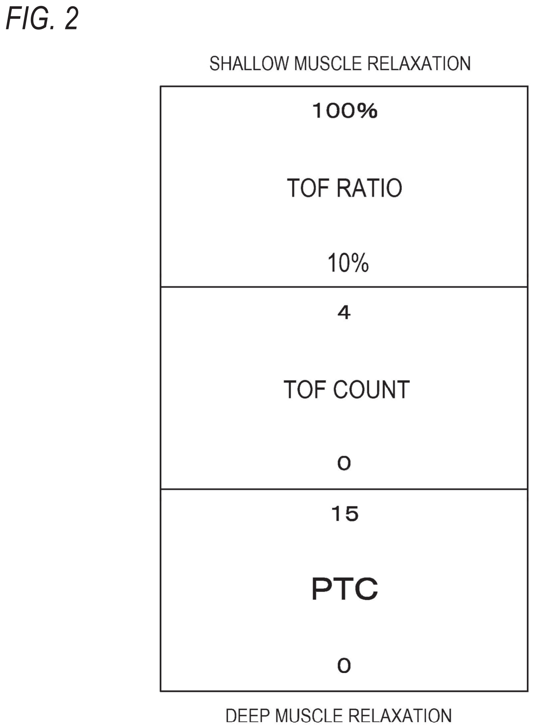

Next, the myoelectric-derived relaxation degree will be described with reference to FIG. 2.

Monitoring of the muscle relaxation degree by the muscle relaxation monitoring device 1 is performed by a train of four (TOF) method in which muscle stimulation is repeated four times every 0.5 seconds and the muscle stimulation is repeated four times every 15 seconds. The muscle stimulation to the patient performed by the TOF method (hereinafter, referred to as "TOF stimulation") is performed at a predetermined stimulation intensity. A value of the stimulation current in the TOF stimulation constitutes a "predetermined current value" in the presently disclosed subject matter, and a value of a stimulation pulse width in the TOF stimulation constitutes a "predetermined pulse width value" in the presently disclosed subject matter.

As illustrated in FIG. 2, examples of the myoelectric-derived relaxation degree can include a TOF ratio, a TOF count, and a post tetanic count (PTC).

A TOF ratio indicates a ratio between an amplitude of the physiological signal by a stimulation of the first time and an amplitude of the physiological signal by a stimulation of the fourth time when the controller 10 stimulates the nerve of the patient four consecutive times every 0.5 seconds via the stimulator 8. Here, when the muscle relaxant is not effective, the amplitude of the physiological signal by the stimulation of the first time and the amplitude of the physiological signal by the stimulation of the fourth time are substantially the same value, and the TOF ratio is a value close to 100%. On the other hand, when the muscle relaxant is effective, the amplitude of the physiological signal by the stimulation of the fourth time is attenuated to a value close to 0. Then, the ratio between the amplitude of the physiological signal by the stimulation of the first time and the amplitude of the physiological signal by the stimulation of the fourth time becomes a value close to 0%. Note that the TOF ratio indicates a shallower lever of the muscle relaxation degree compared to the TOF count and the PTC.

The TOF count indicates the number of counts of physiological signals, that is, the number of counts of waveforms, generated by four consecutive stimulations when the controller 10 stimulates the nerve of the patient four times every 0.5 seconds via the stimulator 8. Here, since the number of counts of the TOF count is the number of counts of the physiological signal when the stimulations of four consecutive times are performed, the number of counts is any value of 0 to 4. Note that the TOF count indicates a deeper level of the muscle relaxation degree compared to the TOF ratio, but a shallower level of the muscle relaxation degree compared to the PTC.

The PTC indicates the number of counts of physiological signals generated, that is, the number of counts of waveforms when the controller 10 stimulates the nerve of the patient 250 times in 5 seconds via the stimulator 8 and the stimulation electrode 2. Here, the number of counts of the PTC is the number of counts of the physiological signal when the stimulation is performed 250 times, and FIG. 2 illustrates a case where the number of counts of the physiological signal is 0 to 15. Note that the PTC indicates a deeper level of the muscle relaxation degree compared to the TOF ratio and TOF count.

### Flowchart of Process Performed by Muscle Relaxation Monitoring Device 1

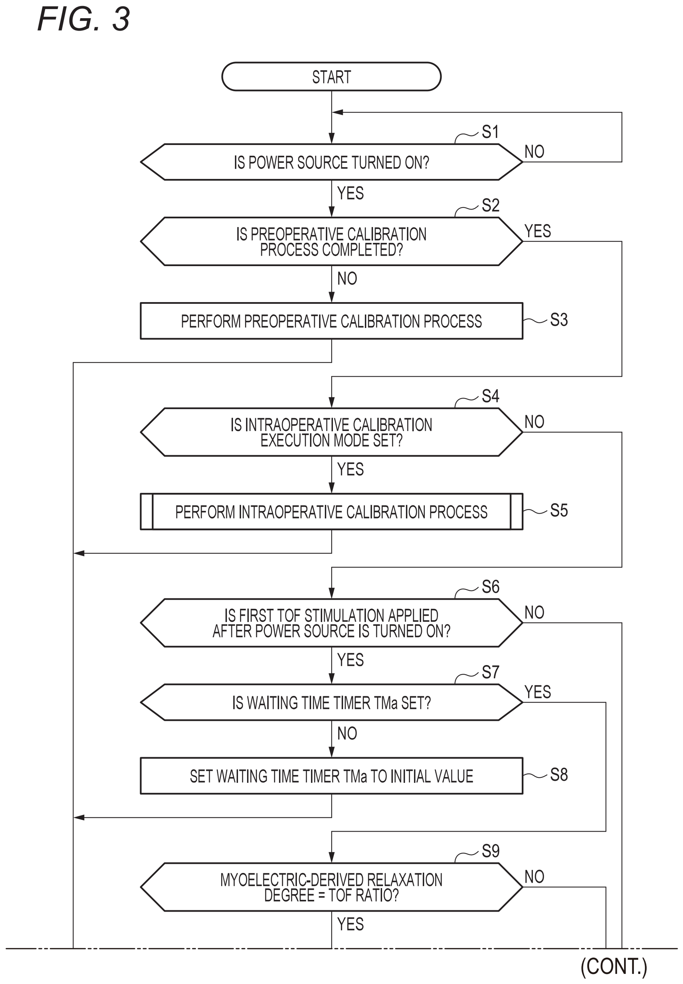

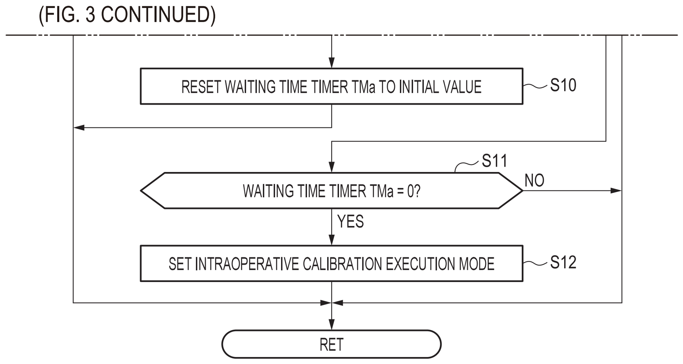

Next, a flowchart of a process performed by the muscle relaxation monitoring device 1 will be described with reference to FIG. 3. The process performed by the muscle relaxation monitoring device 1 is performed at predetermined intervals.

In step S1, the controller 10 performs a process of determining whether a power source of the muscle relaxation monitoring device 1 is turned on. When it is determined that the power source of the muscle relaxation monitoring device 1 is turned on (step S1 = YES), the process proceeds to step S2. On the other hand, when it is determined that the power source of the muscle relaxation monitoring device 1 is not turned on (step S1 = NO), the process of step S1 is repeatedly performed until the power source of the muscle relaxation monitoring device 1 is turned on.

In step S2, the controller 10 performs the process of determining whether a preoperative calibration process is completed. When it is determined that the preoperative calibration process is completed (step S2 = YES), the process proceeds to step S4. On the other hand, when it is determined that the preoperative calibration process is not completed (step S2 = NO), the process proceeds to step S3.

In step S3, the controller 10 performs the preoperative calibration process. Here, by performing the preoperative calibration process, the value of the stimulation current in the TOF stimulation performed thereafter is set to an optimum value. When the process of step S3 ends, the process performed by the muscle relaxation monitoring device 1 ends.

In step S4, the controller 10 performs a process of determining whether an intraoperative calibration execution mode is set. Specifically, the controller 10 performs the process of determining whether the intraoperative calibration execution mode is set with reference to a predetermined storage area of the memory 12. When it is determined that the intraoperative calibration execution mode is set (step S4 = YES), the process proceeds to step S5. On the other hand, when it is determined that the intraoperative calibration execution mode is not set (step S4 = NO), the process proceeds to step S6.

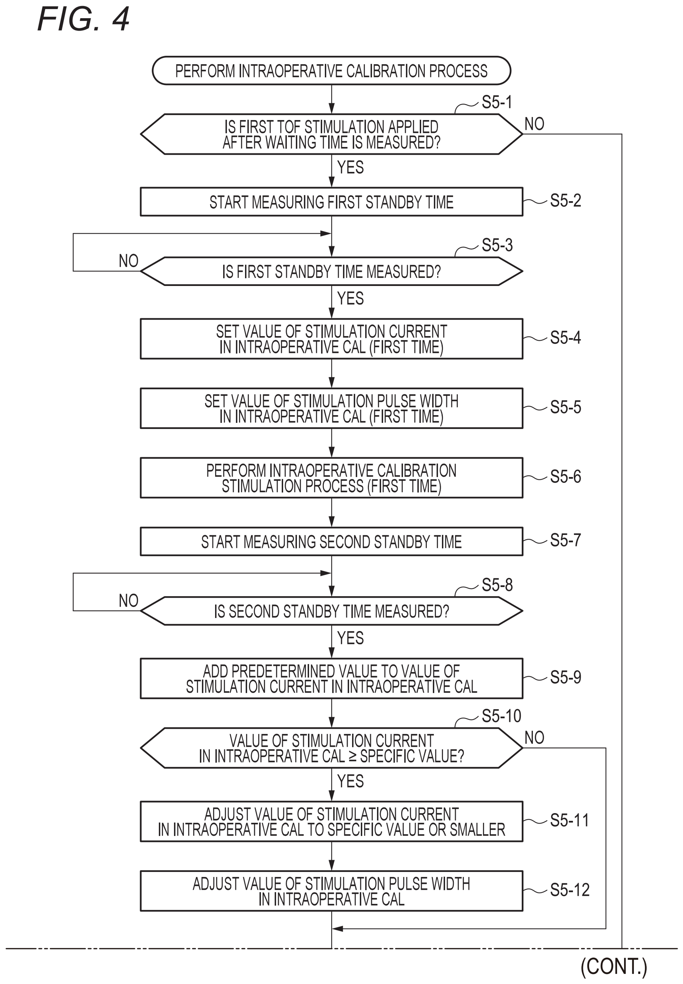

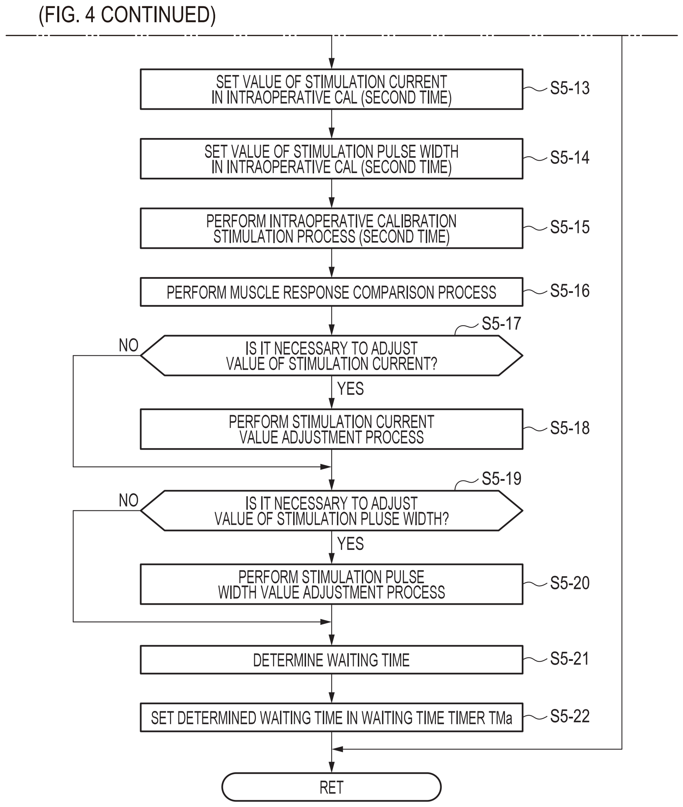

In step S5, the controller 10 performs the intraoperative calibration process which will be specifically described later with reference to FIG. 4. When the process of step S5 ends, the process performed by the muscle relaxation monitoring device 1 ends.

In step S6, the controller 10 performs a process of determining whether the first TOF stimulation is applied after the power source of the muscle relaxation monitoring device 1 is turned on. Then, when it is determined that the first TOF stimulation is applied after the power source of the muscle relaxation monitoring device 1 is turned on (step S6 = YES), the process proceeds to step S7. On the other hand, when it is determined that the first TOF stimulation is not applied after the power source of the muscle relaxation monitoring device 1 is turned on (step S6 = NO), the process performed by the muscle relaxation monitoring device 1 ends.

In step S7, the controller 10 performs a process of determining whether the waiting time timer TMa is set with reference to the value of the waiting time timer TMa. When it is determined that the waiting time timer is set (step S7 = YES), the process proceeds to step S9. On the other hand, when it is determined that the waiting time timer is not set (step S7 = NO), the process proceeds to step S8.

In step S8, the controller 10 performs a process of setting an initial value to the waiting time timer TMa. As a result, the measurement of the waiting time is started. Here, in the present embodiment, the initial value set in the waiting time timer TMa is 30 minutes, but other values can be appropriately set. When the process of step S8 ends, the process performed by the muscle relaxation monitoring device 1 ends.

In step S9, the controller 10 performs a process of determining whether the myoelectric-derived relaxation degree is a TOF ratio. Specifically, the controller 10 performs the process of determining whether the current myoelectric-derived relaxation degree is the TOF ratio as a result of applying the TOF stimulation to the patient. When it is determined that the myoelectric-derived relaxation degree is the TOF ratio (step S9 = YES), the process proceeds to step S10. On the other hand, when it is determined that the myoelectric-derived relaxation degree is not the TOF ratio (step S9 = NO), the process proceeds to step S11.

In step S10, the controller 10 performs a process of resetting the initial value to the waiting time timer TMa. As a result, the measurement of the waiting time is started again. Here, in the present embodiment, the value reset to the waiting time timer TMa is set to the initial value (30 minutes), but other values can be appropriately set. When the process of step S10 ends, the process performed by the muscle relaxation monitoring device 1 ends.

In step S11, the controller 10 performs a process of determining whether the value of the waiting time timer TMa is 0. Thus, a process of determining whether the waiting time is measured is performed. When it is determined that the value of the waiting time timer TMa is 0 (step S11 = YES), the process proceeds to step S12. On the other hand, when it is determined that the value of the waiting time timer TMa is not 0 (step S11 = NO), the process performed by the muscle relaxation monitoring device 1 ends.

In step S12, the controller 10 performs a process of setting the intraoperative calibration execution mode. Specifically, the controller 10 performs the process of setting the intraoperative calibration execution mode in a predetermined storage area of the memory 12. When the process of step S12 ends, the process performed by the muscle relaxation monitoring device 1 ends.

### Subroutine of Intraoperative Calibration Process

Next, a subroutine of the intraoperative calibration process performed in step S5 of the process performed by the muscle relaxation monitoring device 1 will be described with reference to FIG. 4.

In step S5-1, the controller 10 performs a process of determining whether the first TOF stimulation is applied after the waiting time is measured. Specifically, the controller 10 performs the process of determining whether the first TOF stimulation is applied to the patient after the value of the waiting time timer TMa set by the process of step S8, the process of step S10, and the process of step S5-22 to be described later becomes 0. When it is determined that the first TOF stimulation is applied after the waiting time is measured (step S5-1 = YES), the process proceeds to step S5-2. On the other hand, when it is determined that the first TOF stimulation is not applied after the waiting time is measured (step S5-1 = NO), the subroutine of the intraoperative calibration process ends.

In step S5-2, the controller 10 performs a process of starting measuring the first standby time. Specifically, the measurement of the first standby time is started by counting up the value of the first standby time timer TMb. Here, in the present embodiment, the first standby time is set to 10 seconds, but other values can be appropriately set. When the process of step S5-2 ends, the process proceeds to step S5-3.

In step S5-3, the controller 10 performs a process of determining whether the first standby time is measured. Specifically, the controller 10 performs a process of determining whether the value of the first standby time timer TMb is 10 seconds, thereby performing the process of determining whether the first standby time is measured. When it is determined that the first standby time is measured (step S5-3 = YES), the process proceeds to step S5-4. On the other hand, when it is determined that the first standby time is not measured (step S5-3 = NO), the process of step S5-3 is repeatedly performed until the first standby time is measured.

In step S5-4, the controller 10 performs a process of setting the value of the stimulation current in the intraoperative calibration (first time). Specifically, the controller 10 performs the process of setting the value of the stimulation current in the TOF stimulation performed immediately before as the value of the stimulation current in the intraoperative calibration (first time). When the process of step S5-4 ends, the process proceeds to step S5-5.

In step S5-5, the controller 10 performs a process of setting the value of the stimulation pulse width in the intraoperative calibration (first time). Specifically, the controller 10 performs the process of setting the value of the stimulation pulse width in the TOF stimulation performed immediately before as the value of the stimulation pulse width in the intraoperative calibration (first time). When the process of step S5-5 ends, the process proceeds to step S5-6.

In step S5-6, the controller 10 performs the intraoperative calibration stimulation process (first time). Specifically, the controller 10 performs the process of applying stimulation at a stimulation intensity having the value of the stimulation current set by the process of step S5-4 and the value of the stimulation pulse width set by the process of step S5-5. When the process of step S5-6 ends, the process proceeds to step S5-7.

In step S5-7, the controller 10 performs a process of starting measuring the second standby time. Specifically, the measurement of the second standby time is started by counting up the value of the second standby time timer TMc. Here, in the present embodiment, the second standby time is set to 10 seconds, but other values can be appropriately set. When the process of step S5-7 ends, the process proceeds to step S5-8.

In step S5-8, the controller 10 performs a process of determining whether the second standby time is measured. Specifically, the controller 10 performs a process of determining whether the value of the second standby time timer TMc is 10 seconds, thereby performing the process of determining whether the second standby time is measured. When it is determined that the second standby time is measured (step S5-8 = YES), the process proceeds to step S5-9. On the other hand, when it is determined that the second standby time is not measured (step S5-8 = NO), the process of step S5-8 is repeatedly performed until the second standby time is measured.

In step S5-9, the controller 10 performs a process of adding a predetermined value to the value of the stimulation current in the intraoperative calibration. Specifically, the controller 10 performs the process of adding 12 mA to the value of the stimulation current in the intraoperative calibration (first time) set by the process of step S5-4. Here, in the present embodiment, the "predetermined value" is set to 12 mA, but other values can be appropriately set. When the process of step S5-9 ends, the process proceeds to step S5-10.

In step S5-10, the controller 10 performs a process of determining whether the value of the stimulation current in the intraoperative calibration is a value equal to or larger than a specific value. Specifically, as a result of performing the process of step S5-9, the controller 10 performs a process of determining whether the value of the stimulation current in the intraoperative calibration is a value equal to or larger than the specific value, which is the ceiling. Here, in the present embodiment, the "specific value" is 48 mA, but other values can be appropriately set. When it is determined that the value of the stimulation current is a value equal to or larger than the specific value (step S5-10 = YES), the process proceeds to step S5-11. On the other hand, when it is determined that the value of the stimulation current is a value not equal to or larger than the specific value (step S5-10 = NO), the process proceeds to step S5-13.

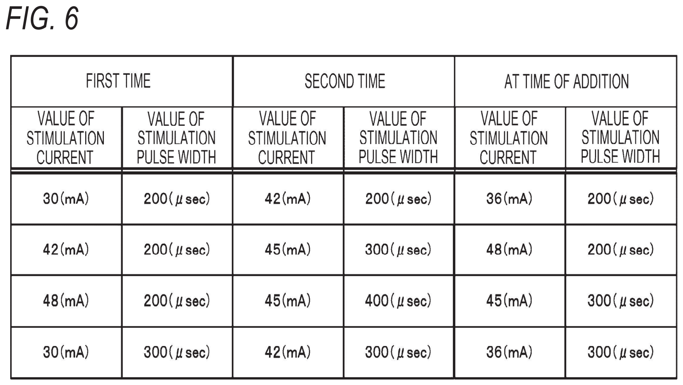

In step S5-11, the controller 10 performs a process of adjusting the value of the stimulation current in the intraoperative calibration to a value equal to or smaller than the specific value. Here, a specific example of adjusting the value of the stimulation current in the preoperative calibration will be described in detail later with reference to FIG. 6. When the process of step S5-11 ends, the process proceeds to step S5-12.

In step S5-12, the controller 10 performs a process of adjusting the value of the stimulation pulse width in the intraoperative calibration. Specifically, the controller 10 performs the process of adjusting the value of the stimulation pulse width within a range from an initial value to a maximum value. The initial value of the stimulation pulse width is 200 µsec, but other values can be appropriately set. The maximum value of the stimulation pulse width is set to 500 µsec, but other values can be appropriately set. When the process of step S5-12 ends, the process proceeds to step S5-13.

In step S5-13, the controller 10 performs a process of setting the value of the stimulation current in the intraoperative calibration (second time). Specifically, when it is determined that the value of the stimulation current in the intraoperative calibration is equal to or larger than the specific value (step S5-10 = YES), the controller 10 sets the value adjusted by the process of step S5-11 as the value of the stimulation current in the intraoperative calibration (second time). On the other hand, when it is determined that the value of the stimulation current in the intraoperative calibration is not equal to or larger than the specific value (step S5-10 = NO), a value obtained by adding a predetermined value to the value of the stimulation current in the intraoperative calibration (first time) is set as the value of the stimulation current in the intraoperative calibration (second time). When the process of step S5-13 ends, the process proceeds to step S5-14.

In step S5-14, the controller 10 performs a process of setting the value of the stimulation pulse width in the intraoperative calibration (second time). Specifically, when it is determined that the value of the stimulation current in the intraoperative calibration is equal to or larger than the specific value (step S5-10 = YES), the controller 10 performs a process of setting the value of the stimulation pulse width adjusted by the process of step S5-12. When it is determined that the value of the stimulation current in the intraoperative calibration is not equal to or larger than the specific value (step S5-10 = NO), the process of setting the stimulation pulse width set by the process of step S5-5 is performed. When the process of step S5-14 ends, the process proceeds to step S5-15.

In step S5-15, the controller 10 performs the intraoperative calibration stimulation process (second time). Specifically, the controller 10 performs the process of applying stimulation at a stimulation intensity having the value of the stimulation current set by the process of step S5-13 and the value of the stimulation pulse width set by the process of step S5-14. When the process of step S5-15 ends, the process proceeds to step S5-16.

In step S5-16, the controller 10 performs a muscle response comparison process. Specifically, the controller 10 performs the process of comparing a muscle response of the intraoperative calibration stimulation process (first time) in step S5-6 with a muscle response of the intraoperative calibration stimulation process (second time) in step S5-15. When the process of step S5-16 ends, the process proceeds to step S5-17.

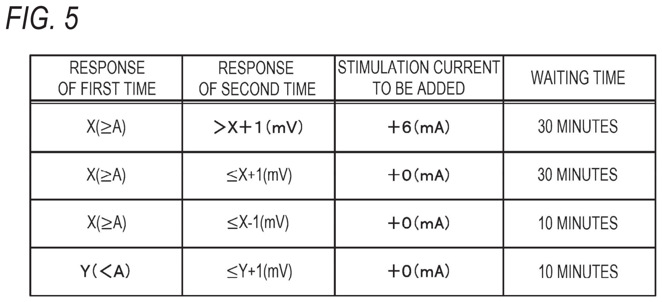

In step S5-17, the controller 10 performs a process of determining whether it is necessary to adjust the value of the stimulation current. Specifically, the controller 10 performs the process of determining whether it is necessary to adjust the value of the stimulation current as a result of performing the muscle response comparison process in step S5-16. Here, whether it is necessary to adjust the value of the stimulation current will be described in detail later with reference to FIG. 5. When it is determined that it is necessary to adjust the value of the stimulation current (step S5-17 = YES), the process proceeds to step S5-18. On the other hand, when it is determined that it is not necessary to adjust the value of the stimulation current (step S5-17 = NO), the process proceeds to step S5-19.

In step S5-18, the controller 10 performs a stimulation current value adjustment process. In this process, the controller 10 performs a process of adjusting the value of the stimulation current in the TOF stimulation performed after the intraoperative calibration process is completed. Here, a specific example of adjusting the value of the stimulation current in the preoperative calibration will be described in detail later with reference to FIG. 6. In the stimulation current value adjustment process in step S5-18, when the value of the stimulation current is adjusted, the value is adjusted to the value equal to or smaller than the specific value. When the process of step S5-18 ends, the process proceeds to step S5-19.

In step S5-19, the controller 10 performs a process of determining whether it is necessary to adjust the value of the stimulation pulse width. Specifically, the controller 10 performs the process of determining whether it is necessary to adjust the value of the stimulation pulse width as a result of performing the muscle response comparison process in step S5-16. Here, whether it is necessary to adjust the value of the stimulation pulse width will be described in detail later with reference to FIG. 5. When it is determined that it is necessary to adjust the value of the stimulation pulse width (step S5-19 = YES), the process proceeds to step S5-20. On the other hand, when it is determined that it is not necessary to adjust the value of the stimulation pulse width (step S5-19 = NO), the process proceeds to step S5-21.

In step S5-20, the controller 10 performs a stimulation pulse width value adjustment process. In this process, the controller 10 performs a process of adjusting the value of the stimulation pulse width in the TOF stimulation performed after the intraoperative calibration process is completed. Here, a specific example of the stimulation pulse width adjusted by the stimulation pulse width value adjustment process will be described in detail later with reference to FIG. 6. In the stimulation pulse width value adjustment process in step S5-20, when the value of the stimulation pulse width is adjusted, the value is adjusted within the range from the initial value to the maximum value. When the process of step S5-20 ends, the process proceeds to step S5-21.

In step S5-21, the controller 10 performs a process of determining a waiting time. A specific example of determining the waiting time will be described later in detail with reference to FIG. 5. When the process of step S5-21 ends, the process proceeds to step S5-22.

In step S5-22, the controller 10 performs a process of setting the determined waiting time in the waiting time timer TMa. Specifically, the controller 10 performs the process of setting the waiting time determined by the process of step S5-21 in the waiting time timer TMa. When the process of step S5-22 ends, the subroutine of the intraoperative calibration process ends.

### Relationship Between Value of Stimulation Current to be Added and Waiting Time

Next, a relationship between the value of the stimulation current to be added and the waiting time will be described with reference to FIG. 5.

A column of a "response of first time" in FIG. 5 indicates a muscle response based on stimulation for the patient in the intraoperative calibration stimulation process (first time) in step S5-6. A column of a "response of second time" in FIG. 5 indicates a muscle response based on the stimulation for the patient in the intraoperative calibration stimulation process (second time) in step S5-15. A column of "stimulation current to be added" in FIG. 5 indicates a value to be added to the value of the stimulation current in the TOF stimulation after the intraoperative calibration process is completed. A column of "waiting time" in FIG. 5 indicates a value set in the waiting time timer TMa, in other words, a time until the intraoperative calibration process is performed next.

As illustrated in a first row of FIG. 5, when the muscle response in response to the intraoperative calibration stimulation process (first time) in step S5-6 is X (≥ a threshold value A) and the muscle response in response to the intraoperative calibration stimulation process (second time) in step S5-15 is a value larger than X + 1 mV, it is determined that the stimulation current is insufficient, and an adjustment value is added. Here, the adjustment value in the present embodiment is 6 mA, but other values can be appropriately set. When the adjustment value is added to the value of the stimulation current, it is determined that it is not necessary to adjust the value of the stimulation current for a while. Therefore, the intraoperative calibration process to be performed next is performed after 30 minutes. Therefore, 30 minutes is determined as the waiting time (step S5-21), and the determined waiting time is set in the waiting time timer TMa (step S5-22).

As illustrated in a second row of FIG. 5, when the muscle response in response to the intraoperative calibration stimulation process (first time) in step S5-6 is X (≥ the threshold value A) and the muscle response in response to the intraoperative calibration stimulation process (second time) in step S5-15 is a value of X + 1 mV or smaller, it is determined that the stimulation current is sufficient, and the value of the stimulation current is not adjusted. When it is determined that the stimulation current is sufficient, it is determined that it is not necessary to adjust the value of the stimulation current for a while. Therefore, the intraoperative calibration process to be performed next is performed after 30 minutes. Therefore, 30 minutes is determined as the waiting time (step S5-21), and the determined waiting time is set in the waiting time timer TMa (step S5-22).

As illustrated in a third row of FIG. 5, when the muscle response in response to the intraoperative calibration stimulation process (first time) in step S5-6 is X (≥ the threshold value A) and the muscle response in response to the intraoperative calibration stimulation process (second time) in step S5-15 is a value of X - 1 mV or smaller, it is determined that there is a muscle response in the intraoperative calibration stimulation process (first time), but there is no muscle response in the intraoperative calibration stimulation process (second time) or the muscle response is significantly lower than that in the intraoperative calibration stimulation process (first time), and it is determined to be an irregular case. The cause of the irregular case is, for example, mixing of noise, and when it is determined that the irregular case occurs, the value of the stimulation current is not adjusted. When it is determined that the current case is the irregular case, the intraoperative calibration process to be performed next is performed after 10 minutes. Therefore, 10 minutes is determined as the waiting time (step S5-21), and the determined waiting time is set in the waiting time timer TMa (step S5-22). Note that the reason why the intraoperative calibration process to be performed next is performed 10 minutes later in the case illustrated in the third row of FIG. 5 is that the waiting time until the intraoperative calibration process is performed next after 30 minutes is too long even though it is determined to be the irregular case.

As illustrated in a fourth row of FIG. 5, when the muscle response in response to the intraoperative calibration stimulation process (first time) in step S5-6 is Y (< the threshold value A) and the muscle response in response to the intraoperative calibration stimulation process (second time) in step S5-15 is a value of Y + 1 mV or smaller, it is determined that the muscle relaxant administered to the patient is effective and the TOF count is 0. In this case, the adjustment value is not added. When it is determined that the TOF count is 0, the intraoperative calibration process to be performed next is performed after 10 minutes. Therefore, 10 minutes is determined as the waiting time (step S5-21), and the determined waiting time is set in the waiting time timer TMa (step S5-22). The reason why the intraoperative calibration process to be performed next is performed after 10 minutes in the case illustrated in the fourth row in FIG. 5 is that the intraoperative calibration process to be performed next is after 30 minutes, which is too long.

### Example of Stimulation Intensity

Next, an example of the stimulation intensity will be described with reference to FIG. 6.

In a column of the "first time" in FIG. 6, the value of the stimulation current stimulated in the intraoperative calibration stimulation process (first time) in step S5-6 and the value of the stimulation pulse width are illustrated. The value of the stimulation current in the column of the "first time" in FIG. 6 is an example of a "first current value" in the presently disclosed subject matter, and the value of the stimulation pulse width in the column of the "first time" in FIG. 6 is an example of a "first pulse width value" in the presently disclosed subject matter. The product of the value of the stimulation current in the column of the "first time" in FIG. 6 and the value of the stimulation pulse width in the column of the "first time" in FIG. 6 is a "first stimulation" in the presently disclosed subject matter.

In a column of the "second time" in FIG. 6, the value of the stimulation current stimulated in the intraoperative calibration stimulation process (second time) in step S5-15 and the value of the stimulation pulse width are illustrated. The value of the stimulation current in the column of the "second time" in FIG. 6 is an example of a "second current value" in the presently disclosed subject matter, and the value of the stimulation pulse width in the column of the "second time" in FIG. 6 is an example of a "second pulse width value" in the presently disclosed subject matter. The product of the value of the stimulation current in the column of the "second time" in FIG. 6 and the value of the stimulation pulse width in the column of the "second time" in FIG. 6 is a "second stimulation" in the presently disclosed subject matter.

In a column of "at the time of addition" in FIG. 6, the value of the stimulation current and the value of the stimulation pulse width in the TOF stimulation after the intraoperative calibration process are illustrated. The value of the stimulation current in the TOF stimulation performed after the intraoperative calibration process is the value of the stimulation current stimulated in the intraoperative calibration stimulation process (first time) to be performed next. Same or similarly, the value of the stimulation pulse width in the TOF stimulation performed after the intraoperative calibration process is the value of the stimulation pulse width stimulated in the intraoperative calibration stimulation process (first time) to be performed next.

First, as illustrated in a first row of FIG. 6, in the intraoperative calibration process (first time), it is assumed that stimulation is performed at a stimulation intensity with a value of the stimulation current of 30 mA and a value of the stimulation pulse width of 200 µsec. Subsequently, in the intraoperative calibration process (second time), when 12 mA is added to the value of the stimulation current in the intraoperative calibration, the value of the stimulation current becomes 30 mA + 12 mA = 42 mA (step S5-9). At this time, since the value of the stimulation current in the intraoperative calibration is not equal to or larger than the specific value of 48 mA (step S5-10 = NO), the value of the stimulation current in the intraoperative calibration is set to 42 mA (step S5-13), and the value of the stimulation pulse width in the intraoperative calibration is set to 200 µsec (step S5-14). Therefore, in the intraoperative calibration process (second time), stimulation is performed at a stimulation intensity with a value of the stimulation current of 42 mA and a value of the stimulation pulse width of 200 µsec (step S5-15).

Then, as a result of comparing the muscle response of the intraoperative calibration stimulation process (first time) with the muscle response of the intraoperative calibration stimulation process (second time) (step S5-16), when it is determined that it is necessary to adjust the value of the stimulation current (step S5-17 = YES), a process of adding the adjustment value (6 mA) to the value (30 mA) of the stimulation current in the intraoperative calibration process (first time) and adjusting the value is performed. As a result, in the TOF stimulation performed after the intraoperative calibration process is completed, stimulation is performed at a stimulation intensity with a value of the stimulation current of 36 mA and a value of the stimulation pulse width of 200 µsec.

As described above, as a result of comparing the muscle response obtained by the intraoperative calibration stimulation process (first time) with the muscle response obtained by the intraoperative calibration stimulation process (second time), the value (36 mA) of the stimulation current in the TOF stimulation performed thereafter is adjusted to a value equal to or larger than the value (30 mA) of the stimulation current in the intraoperative calibration stimulation process (first time), and the value (200 µsec) of the stimulation pulse width in the TOF stimulation performed thereafter is adjusted to a value equal to or larger than the value (200 µsec) of the stimulation pulse width in the intraoperative calibration stimulation process (first time).

As a result of comparing the muscle response obtained by the intraoperative calibration stimulation process (first time) with the muscle response obtained by the intraoperative calibration stimulation process (second time), the value (36 mA) of the stimulation current in the TOF stimulation performed thereafter is adjusted to a value equal to or smaller than the value (42 mA) of the stimulation current in the intraoperative calibration stimulation process (second time), and the value (200 µsec) of the stimulation pulse width in the TOF stimulation performed thereafter is adjusted to a value equal to or smaller than the value (200 µsec) of the stimulation pulse width in the intraoperative calibration stimulation process (second time).

Next, as illustrated in a second row of FIG. 6, in the intraoperative calibration process (first time), it is assumed that the patient is stimulated at a stimulation intensity with a value of the stimulation current of 42 mA and a value of the stimulation pulse width of 200 µsec. Subsequently, in the intraoperative calibration process (second time), when 12 mA is added to the value of the stimulation current in the intraoperative calibration, the value of the stimulation current becomes 42 mA + 12 mA = 54 mA (step S5-9). At this time, since the value of the stimulation current in the intraoperative calibration is equal to or larger than the specific value of 48 mA (step S5-10 = YES), a process of adjusting the value of the stimulation current in the intraoperative calibration to 48 mA or smaller is performed (step S5-11), and a process of adjusting the value of the stimulation pulse width in the intraoperative calibration is performed (step S5-12). Thus, the value of the stimulation current in the intraoperative calibration is set to 45 mA (step S5-13), and the value of the stimulation pulse width in the intraoperative calibration is set to 300 µsec (step S5-14). Therefore, in the intraoperative calibration process (second time), stimulation is performed at a stimulation intensity with a value of the stimulation current of 45 mA and a value of the stimulation pulse width of 300 µsec (step S5-15).

Then, as a result of comparing the muscle response in the intraoperative calibration stimulation process (first time) with the muscle response in the intraoperative calibration stimulation process (second time) in step S5-15 (step S5-16), when it is determined that it is necessary to adjust the value of the stimulation current (step S5-17 = YES), a process of adding the adjustment value (6 mA) to the value (42 mA) of the stimulation current in the intraoperative calibration process (first time) is performed. As a result, in the TOF stimulation performed after the intraoperative calibration process is completed, stimulation is performed at a stimulation intensity with a value of the stimulation current of 48 mA and a value of the stimulation pulse width of 200 µsec.

As described above, as a result of comparing the muscle response obtained by the intraoperative calibration stimulation process (first time) with the muscle response obtained by the intraoperative calibration stimulation process (second time), the value (48 mA) of the stimulation current in the TOF stimulation performed thereafter is adjusted to a value equal to or larger than the value (42 mA) of the stimulation current in the intraoperative calibration stimulation process (first time), and the value (200 µsec) of the stimulation pulse width in the TOF stimulation performed thereafter is adjusted to the value equal to or larger than the value (200 µsec) of the stimulation pulse width in the intraoperative calibration stimulation process (first time).

As a result of comparing the muscle response obtained by the intraoperative calibration stimulation process (first time) with the muscle response obtained by the intraoperative calibration stimulation process (second time), the value (48 mA) of the stimulation current in the TOF stimulation performed thereafter is adjusted to a value equal to or larger than the value (45 mA) of the stimulation current in the intraoperative calibration stimulation process (second time), and the value (200 µsec) of the stimulation pulse width in the TOF stimulation performed thereafter is adjusted to a value equal to or smaller than the value (300 µsec) of the stimulation pulse width in the intraoperative calibration stimulation process (second time).

Next, as illustrated in a third row of FIG. 6, it is assumed that the patient is stimulated at a stimulation intensity with a value of the stimulation current of 48 mA and a value of the stimulation pulse width of 200 µsec in the intraoperative calibration process (first time). Subsequently, in the intraoperative calibration process (second time), when 12 mA is added to the value of the stimulation current in the intraoperative calibration, the value of the stimulation current becomes 48 mA + 12 mA = 60 mA (step S5-9). At this time, since the value of the stimulation current in the intraoperative calibration is equal to or larger than the specific value of 48 mA (step S5-10 = YES), the process of adjusting the value of the stimulation current in the intraoperative calibration to 48 mA or smaller is performed (step S5-11), and the process of adjusting the value of the stimulation pulse width in the intraoperative calibration is performed (step S5-12). Thus, the value of the stimulation current in the intraoperative calibration is set to 45 mA (step S5-13), and the value of the stimulation pulse width in the intraoperative calibration is set to 400 µsec (step S5-14). Therefore, in the intraoperative calibration process (second time), stimulation is performed at a stimulation intensity with a value of the stimulation current of 45 mA and a value of the stimulation pulse width of 400 µsec (step S5-15).

Then, as a result of comparing the muscle response in the intraoperative calibration stimulation process (first time) with the muscle response in the intraoperative calibration stimulation process (second time) in step S5-15 (step S5-16), when it is determined that it is necessary to adjust the value of the stimulation current (step S5-17 = YES), a process of adjusting the value of the stimulation current in the intraoperative calibration process (first time) is performed. As a result, in the TOF stimulation performed after the intraoperative calibration process is completed, stimulation is performed at a stimulation intensity with a value of the stimulation current of 45 mA and a value of the stimulation pulse width of 300 µsec.

Here, as illustrated in the third row of FIG. 6, when the stimulation intensity in the stimulation performed in the intraoperative calibration stimulation process (second time) is adjusted or when the stimulation intensity in the TOF stimulation performed after completion of the intraoperative calibration process is adjusted, as the result, the value of the stimulation current may be smaller than the value of the stimulation current in the intraoperative calibration stimulation process (first time). This is because there is a case where the value of the stimulation current is subtracted and the value of the stimulation pulse width is added due to the fact that the stimulation intensity is calculated by the product of the value of the stimulation current and the value of the stimulation pulse width and the fact that the specific value which is the ceiling of the value of the stimulation current is determined.

Specifically, as illustrated in the third row of FIG. 6, since the value of the stimulation current of the stimulation performed in the intraoperative calibration stimulation process (first time) is already 48 mA which is the specific value, the value of the stimulation current cannot be added any more. Therefore, when the stimulation intensity of the stimulation performed in the intraoperative calibration stimulation process (second time) is adjusted or when the stimulation intensity in the TOF stimulation performed after the completion of the intraoperative calibration process is adjusted, it is necessary to add the value of the stimulation pulse width.

At this time, instead of simply adding and adjusting the value of the stimulation pulse width, adjustment is performing by adding the value of the stimulation pulse width and subtracting the value of the stimulation current. Specifically, when the stimulation intensity of the stimulation performed in the intraoperative calibration stimulation process (second time) is adjusted, 3 mA is subtracted from the value of the stimulation current of the stimulation performed in the intraoperative calibration stimulation process (first time) (step S5-11), and 200 µsec is added to the value of the stimulation pulse width of the stimulation performed in the intraoperative calibration stimulation process (first time) (step S5-12).

Same or similarly, when the stimulation intensity in the TOF stimulation performed after the completion of the intraoperative calibration process is adjusted, 3 mA is subtracted from the value of the stimulation current in the stimulation performed in the intraoperative calibration stimulation process (first time) (step S5-18), and 100 µsec is added to the value of the stimulation pulse width in the stimulation performed in the intraoperative calibration stimulation process (first time) (step S5-20).

In the present embodiment, the value of the stimulation pulse width is adjusted in units of 100 µsec, but for example, even if 100 µsec is added to the value of the stimulation pulse width, adjustment of simply subtracting a constant value from the value of the stimulation current is not performed. This is because, even when the stimulation intensity is the same (for example, 12000), it is considered that the stimulation intensity is higher when the value of the stimulation current is large (for example, 40 mA) and the value of the stimulation pulse width is small (for example, 300 µsec) than when the value of the stimulation current is small (for example, 30 mA) and the value of the stimulation pulse width is large (for example, 400 µsec). As a result, the stimulation performed in the intraoperative calibration process (second time) and the TOF stimulation performed after the completion of the intraoperative calibration process can be adjusted to optimum stimulation intensities.

As described above, as a result of comparing the muscle response obtained by the intraoperative calibration stimulation process (first time) with the muscle response obtained by the intraoperative calibration stimulation process (second time), the value (45 mA) of the stimulation current in the TOF stimulation performed thereafter is adjusted to a value equal to or smaller than the value (45 mA) of the stimulation current in the intraoperative calibration stimulation process (second time), and the value (300 µsec) of the stimulation pulse width in the TOF stimulation performed thereafter is adjusted to a value equal to or smaller than the value (400 µsec) of the stimulation pulse width in the intraoperative calibration stimulation process (second time).

As a result of comparing the muscle response obtained by the intraoperative calibration stimulation process (first time) with the muscle response obtained by the intraoperative calibration stimulation process (second time), the value (45 mA) of the stimulation current in the TOF stimulation performed thereafter is adjusted to a value equal to or smaller than the value (48 mA) of the stimulation current in the intraoperative calibration stimulation process (first time), and the value (300 µsec) of the stimulation pulse width in the TOF stimulation performed thereafter is adjusted to the value equal to or larger than the value (200 µsec) of the stimulation pulse width in the intraoperative calibration stimulation process (first time).

As a result of comparing the muscle response obtained by the intraoperative calibration stimulation process (first time) with the muscle response obtained by the intraoperative calibration stimulation process (second time), the value (45 mA) of the stimulation current in the TOF stimulation performed thereafter is adjusted to the value equal to or larger than the value (45 mA) of the stimulation current in the intraoperative calibration stimulation process (second time), and the value (300 µsec) of the stimulation pulse width in the TOF stimulation performed thereafter is adjusted to the value equal to or smaller than the value (400 µsec) of the stimulation pulse width in the intraoperative calibration stimulation process (second time).

Next, as illustrated in a fourth row of FIG. 6, it is assumed that the patient is stimulated at a stimulation intensity with a value of the stimulation current of 30 mA and a value of the stimulation pulse width of 300 µsec in the intraoperative calibration process (first time). Subsequently, in the intraoperative calibration process (second time), when 12 mA is added to the value of the stimulation current in the intraoperative calibration, the value of the stimulation current becomes 30 mA + 12 mA = 42 mA (step S5-9). At this time, since the value of the stimulation current in the intraoperative calibration is not equal to or larger than the specific value of 48 mA (step S5-10 = NO), the value of the stimulation current in the intraoperative calibration is set to 42 mA (step S5-13), and the value of the stimulation pulse width in the intraoperative calibration is set to 300 µsec (step S5-14). Therefore, in the intraoperative calibration process (second time), stimulation is performed at a stimulation intensity with a value of the stimulation current of 42 mA and a value of the stimulation pulse width of 300 µsec (step S5-15).

Then, as a result of comparing the muscle response in the intraoperative calibration stimulation process (first time) with the muscle response in the intraoperative calibration stimulation process (second time) in step S5-15 (step S5-16), when it is determined that it is necessary to adjust the value of the stimulation current (step S5-17 = YES), a process of adding the adjustment value (6 mA) to the value (30 mA) of the stimulation current of the intraoperative calibration process (first time) is performed. As a result, in the TOF stimulation performed after the intraoperative calibration process is completed, stimulation is performed at a stimulation intensity with a value of the stimulation current value of 36 mA and a value of the stimulation pulse width of 300 µsec.

As described above, as a result of comparing the muscle response obtained by the intraoperative calibration stimulation process (first time) with the muscle response obtained by the intraoperative calibration stimulation process (second time), the value (36 mA) of the stimulation current in the TOF stimulation performed thereafter is adjusted to a value equal to or larger than the value (30 mA) of the stimulation current in the intraoperative calibration stimulation process (first time), and the value (300 µsec) of the stimulation pulse width in the TOF stimulation performed thereafter is adjusted to a value equal to or larger than the value (300 µsec) of the stimulation pulse width in the intraoperative calibration stimulation process (first time).

As a result of comparing the muscle response obtained by the intraoperative calibration stimulation process (first time) with the muscle response obtained by the intraoperative calibration stimulation process (second time), the value (36 mA) of the stimulation current in the TOF stimulation performed thereafter is adjusted to the value equal to or smaller than the value (42 mA) of the stimulation current in the intraoperative calibration stimulation process (second time), and the value (300 µsec) of the stimulation pulse width in the TOF stimulation performed thereafter is adjusted to a value equal to or smaller than the value (300 µsec) of the stimulation pulse width in the intraoperative calibration stimulation process (second time).

Note that the example in the first row in FIG. 6 and the example in the fourth row in FIG. 6 are examples of adjusting only the value of the stimulation current in the TOF stimulation performed after the intraoperative calibration stimulation process (second time) is performed. The example of the second row and the example of the third row in FIG. 6 are examples of adjusting the value of the stimulation current and the value of the stimulation pulse width in the TOF stimulation performed after the intraoperative calibration stimulation process (second time) is performed. Although not illustrated in FIG. 6, only the value of the stimulation pulse width in the TOF stimulation performed after the intraoperative calibration stimulation process (second time) is performed may be adjusted.

### Timing Chart of Intraoperative Calibration

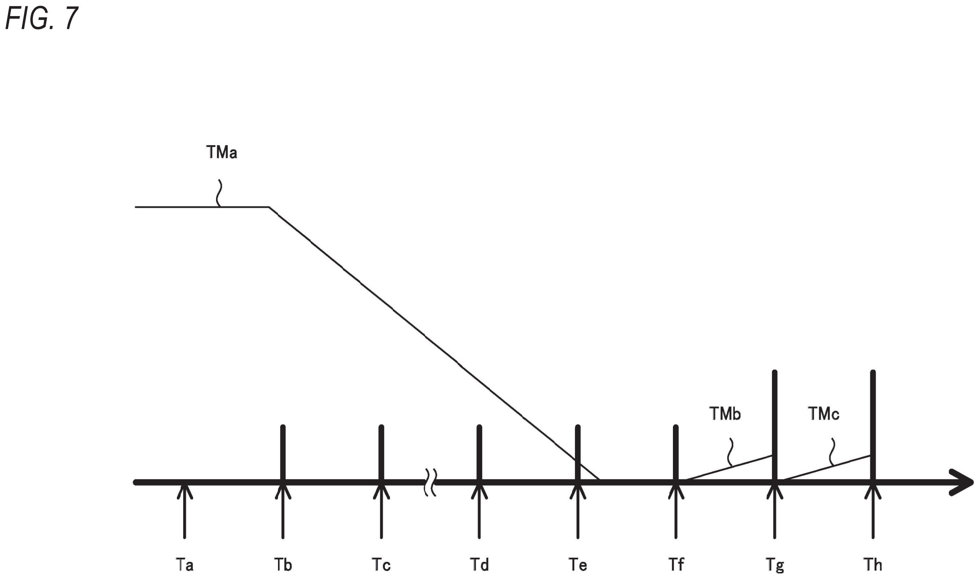

Next, a timing chart of the intraoperative calibration will be described with reference to FIG. 7.

First, a timing Ta in FIG. 7 is a timing at which the preoperative calibration is performed. The preoperative calibration is performed before the muscle relaxant is administered to the patient and before the TOF stimulation is performed.

Next, a timing Tb in FIG. 7 is a timing at which the first TOF stimulation is performed after the power source of the muscle relaxation monitoring device 1 is turned on. At this time, the measurement of the waiting time is started. The waiting time is measured by setting an initial value of 30 minutes to the waiting time timer TMa and counting down the value of the waiting time timer TMa. Here, the reason why the timing at which the measurement of the waiting time is started is set to the timing at which the first TOF stimulation is performed after the power source of the muscle relaxation monitoring device 1 is turned on is that, for example, when the timing at which the measurement of the waiting time is started is set to the timing at which the bedside monitor 20 is started up, there is a possibility that the intraoperative calibration process is suddenly performed when the power source of the bedside monitor 20 is turned on first thing in the morning. On the other hand, when it is a timing that the first TOF stimulation is performed after the power source of the muscle relaxation monitoring device 1 is turned on, the intraoperative calibration process can be performed at an appropriate timing. Note that "the first TOF stimulation is performed after the power source of the muscle relaxation monitoring device 1 is turned on" corresponds to "a predetermined condition is satisfied" in the presently disclosed subject matter, and "the time set in the waiting time timer TMa" corresponds to a "preparation time" in the presently disclosed subject matter.

Next, a timing Tc in FIG. 7 is a timing at which the TOF stimulation of the second time is performed after the power source of the muscle relaxation monitoring device 1 is turned on. A timing Td in FIG. 7 is a timing at which a TOF stimulation of an N-th time is performed after the power source of the muscle relaxation monitoring device 1 is turned on. A timing Te in FIG. 7 is a timing at which the TOF stimulation is performed immediately before the measurement of the waiting time is completed.

Next, a timing Tf in FIG. 7 is a timing at which the TOF stimulation is first performed immediately after the measurement of the waiting time is completed. At this time, measurement of the first standby time (10 seconds) is started. The first standby time is measured by counting up the value of the first standby time timer TMb. The "first standby time" corresponds to a "first time" in the presently disclosed subject matter.

Next, a timing Tg in FIG. 7 is a timing at which the measurement of the first standby time is completed and the intraoperative calibration stimulation process (first time) is performed. At this time, measurement of the second standby time (10 seconds) is started. The second standby time is measured by counting up the value of the second standby time timer TMc. The "second standby time" corresponds to a "second time" in the presently disclosed subject matter.

Here, the timing at which the intraoperative calibration stimulation process (first time) is performed is triggered by the completion of the measurement of the first standby time in order to prevent stimulation from being applied to the patient in a state in which the patient is awake.

Next, a timing Th in FIG. 7 is a timing at which the measurement of the second standby time is completed and the intraoperative calibration stimulation process (second time) is performed. The reason why the timing at which the intraoperative calibration stimulation process (second time) is performed is triggered by the completion of the measurement of the second standby time is the same as the reason why the timing at which the intraoperative calibration stimulation process (first time) is performed is triggered by the completion of the measurement of the first standby time. Thereafter, the muscle response comparison process of comparing the muscle response when the intraoperative calibration stimulation process (first time) is performed with the muscle response when the intraoperative calibration stimulation process (second time) is performed is performed. Then, the stimulation intensity in the TOF stimulation to be performed thereafter is adjusted according to the result of the muscle response comparison process.

### Timing Chart when TOF Ratio is Detected

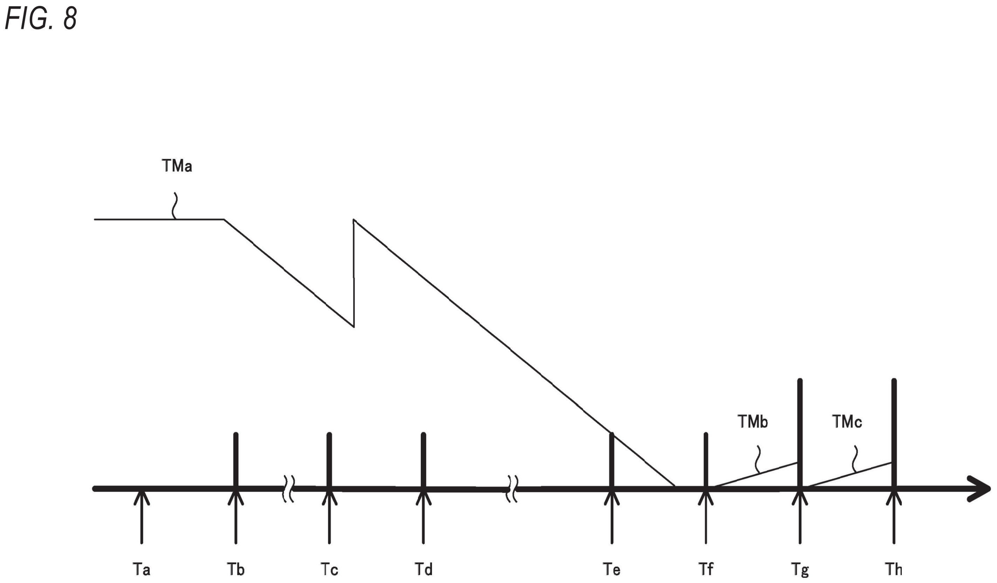

Next, a timing chart when the TOF ratio is detected will be described with reference to FIG. 8.

First, the timing Ta in FIG. 8 is the timing at which the preoperative calibration is performed. Next, the timing Tb in FIG. 8 is the timing at which the first TOF stimulation is performed after the power source of the muscle relaxation monitoring device 1 is turned on. Next, the timing Tc in FIG. 8 is a timing at which the TOF stimulation of the N-th time is performed after the power source of the muscle relaxation monitoring device 1 is turned on. At this time, when the TOF ratio is detected as the myoelectric-derived relaxation degree, since there is a constant amplitude, it is determined that the stimulation current is sufficient, and the initial value of 30 minutes is reset to the waiting time timer TMa. Accordingly, the measurement of the waiting time is started again after the timing Tc in FIG. 8.

The timing Td in FIG. 8 is a timing at which a TOF stimulation of an (N+1)-th time is performed after the power source of the muscle relaxation monitoring device 1 is turned on. Next, the timing Te in FIG. 8 is the timing at which the TOF stimulation is performed immediately before the measurement of the waiting time is completed. The timing Tf in FIG. 8 is the timing at which the TOF stimulation is first performed immediately after the measurement of the waiting time is completed. Next, the timing Tg in FIG. 8 is the timing at which the measurement of the first standby time is completed and the intraoperative calibration stimulation process (first time) is performed. Next, the timing Th in FIG. 8 is the timing at which the measurement of the second standby time is completed and the intraoperative calibration stimulation process (second time) is performed.

In this way, when the TOF ratio is detected as the myoelectric-derived relaxation degree, since there is a constant amplitude, it is determined that the stimulation current is sufficient, and the value of the waiting time timer TMa is reset. Accordingly, the intraoperative calibration process can be performed at an appropriate timing.

### Second Embodiment

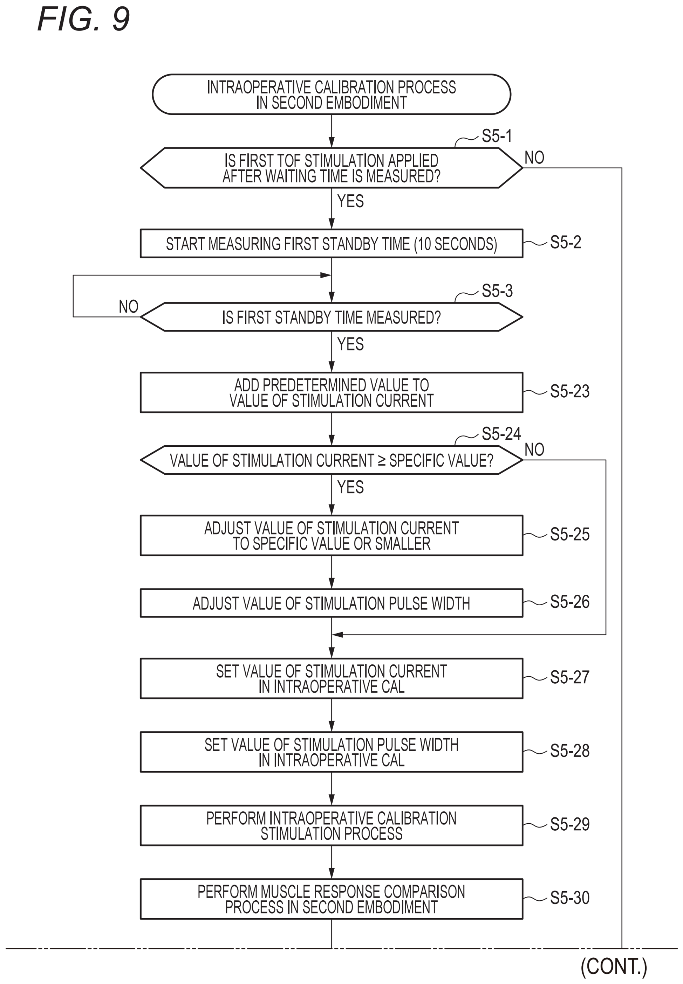

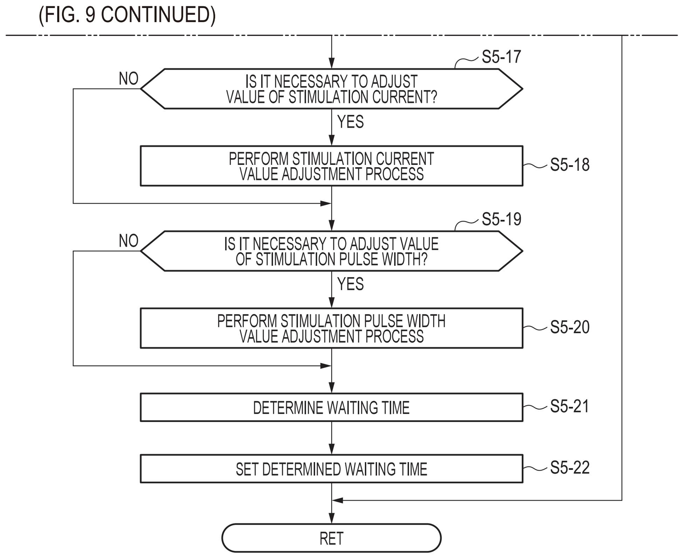

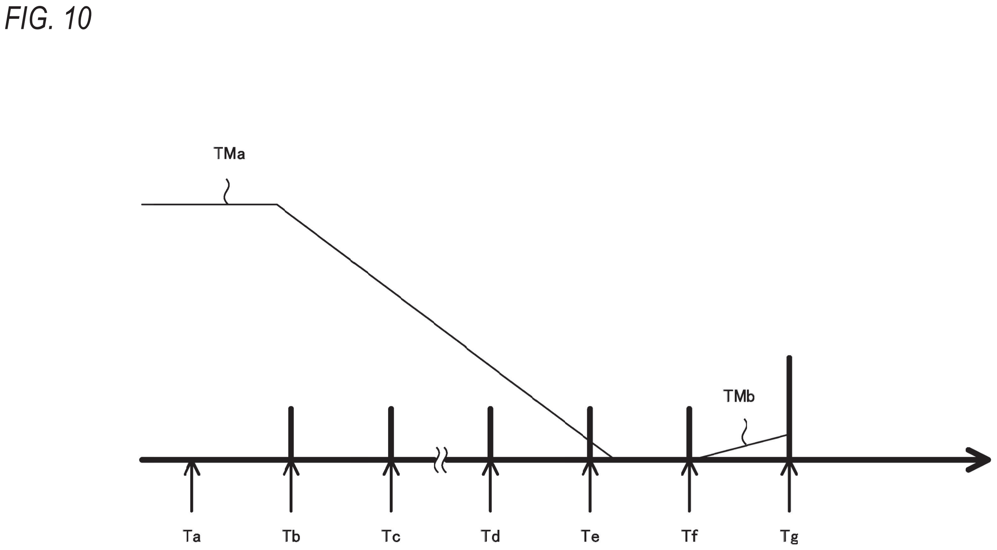

Hereinafter, a second embodiment of the presently disclosed subject matter will be described with reference to FIGS. 9 and 10. In the second embodiment, description of points common to the first embodiment will be omitted, and only points different from the first embodiment will be described.

### Subroutine of Intraoperative Calibration Process in Second Embodiment

A subroutine of the intraoperative calibration process in the second embodiment will be described with reference to FIG. 9.

In step S5-23, the controller 10 performs the process of adding the predetermined value to the value of the stimulation current. Specifically, after the measurement of the first standby time is completed, the controller 10 performs a process of adding 12 mA to the value of the stimulation current in the TOF stimulation performed first. When the process of step S5-23 ends, the process proceeds to step S5-24.

In step S5-24, the controller 10 performs the process of determining whether the value of the stimulation current is the value equal to or larger than the specific value. Specifically, as a result of performing the process of step S5-23, the controller 10 performs a process of determining whether the value in the TOF stimulation is the value equal to or larger than the specific value, which is the ceiling. When it is determined that the value of the stimulation current is the value equal to or larger than the specific value (step S5-24 = YES), the process proceeds to step S5-25. On the other hand, when it is determined that the value of the stimulation current is a value not equal to or larger than the specific value (step S5-24 = NO), the process proceeds to step S5-27.

In step S5-25, the controller 10 performs the process of adjusting the value of the stimulation current to the value equal to or smaller than a specific value. When the process of step S5-25 ends, the process proceeds to step S5-26.

In step S5-26, the controller 10 performs the process of adjusting the value of the stimulation pulse width. Specifically, the controller 10 performs the process of adjusting the value of the stimulation pulse width within the range from the initial value to the maximum value. When the process of step S5-26 ends, the process proceeds to step S5-27.

In step S5-27, the controller 10 performs the process of setting the value of the stimulation current in the intraoperative calibration. Specifically, when the controller 10 determines that the value of the stimulation current is equal to or larger than the specific value (step S5-24 = YES), the value adjusted by the process of step S5-25 is set as the value of the stimulation current in the intraoperative calibration. On the other hand, when it is determined that the value of the stimulation current is not equal to or larger than the specific value (step S5-24 = NO), a value obtained by adding the predetermined value to the value of the stimulation current in the TOF stimulation performed first after the completion of the measurement of the waiting time is set as the value of the stimulation current in the intraoperative calibration. When the process of step S5-27 ends, the process proceeds to step S5-28.

In step S5-28, the controller 10 performs a process of setting the value of the stimulation pulse width in the intraoperative calibration. Specifically, when it is determined that the value of the stimulation current is equal to or larger than the specific value (step S5-24 = YES), the controller 10 performs the process of setting the value of the stimulation pulse width adjusted by the process of step S5-26. When it is determined that the value of the stimulation current is not equal to or larger than the specific value (step S5-24 = NO), a process of setting the value of the stimulation pulse width in the TOF stimulation performed first is performed after the measurement of the waiting time is completed. When the process of step S5-28 ends, the process proceeds to step S5-29.

In step S5-29, the controller 10 performs the intraoperative calibration stimulation process. Specifically, the controller 10 performs a process of applying a stimulation at a stimulation intensity having the value of the stimulation current set by the process of step S5-27 and the value of the stimulation pulse width set by the process of step S5-28. When the process of step S5-29 ends, the process proceeds to step S5-30.

In step S5-30, the controller 10 performs the muscle response comparison process in the second embodiment. Specifically, after the measurement of the waiting time is completed, the controller 10 performs the process of comparing the muscle response in the TOF stimulation performed first with the muscle response in the intraoperative calibration stimulation process in step S5-29. When the process of step S5-30 ends, the process proceeds to step S5-17.

### Timing Chart of Intraoperative Calibration in Second Embodiment

Next, a timing chart of the intraoperative calibration in the second embodiment will be described with reference to FIG. 10.

The timing Ta in FIG. 10 is the timing at which the preoperative calibration is performed. The timing Tb in FIG. 10 is the timing at which the first TOF stimulation is performed after the power source of the muscle relaxation monitoring device 1 is turned on. The timing Tc in FIG. 10 is the timing at which the TOF stimulation of the second time is performed after the power source of the muscle relaxation monitoring device 1 is turned on. The timing Td in FIG. 10 is the timing at which the TOF stimulation of the N-th time is performed after the power source of the muscle relaxation monitoring device 1 is turned on. The timing Te in FIG. 10 is a timing at which the TOF stimulation is performed immediately before the measurement of the waiting time is completed.

The timing Tf in FIG. 10 is a timing at which the first TOF stimulation is performed immediately after the value of the waiting time timer TMa becomes 0 and the measurement of the waiting time is completed. At this time, the measurement of the first standby time is started. The timing Tg in FIG. 10 is the timing at which the measurement of the first standby time is completed and the intraoperative calibration stimulation process is performed. In the muscle response comparison process in the second embodiment performed thereafter, after the measurement of the waiting time is completed, the process of comparing the muscle response in the TOF stimulation performed first with the muscle response in the intraoperative calibration stimulation process (first time) is performed. Then, the stimulation intensity in the TOF stimulation to be performed thereafter is adjusted according to the result of the muscle response comparison process in the second embodiment.

As described above, in the second embodiment, after the measurement of the waiting time is completed, the stimulation intensity in the TOF stimulation performed thereafter is adjusted according to the result of the comparing the muscle response in the TOF stimulation performed first and the muscle response in the intraoperative calibration stimulation process. This eliminates the need to perform the intraoperative calibration stimulation process a plurality of times, thereby reducing a process load of the intraoperative calibration process as compared with the intraoperative calibration process in the first embodiment.

### Third Embodiment

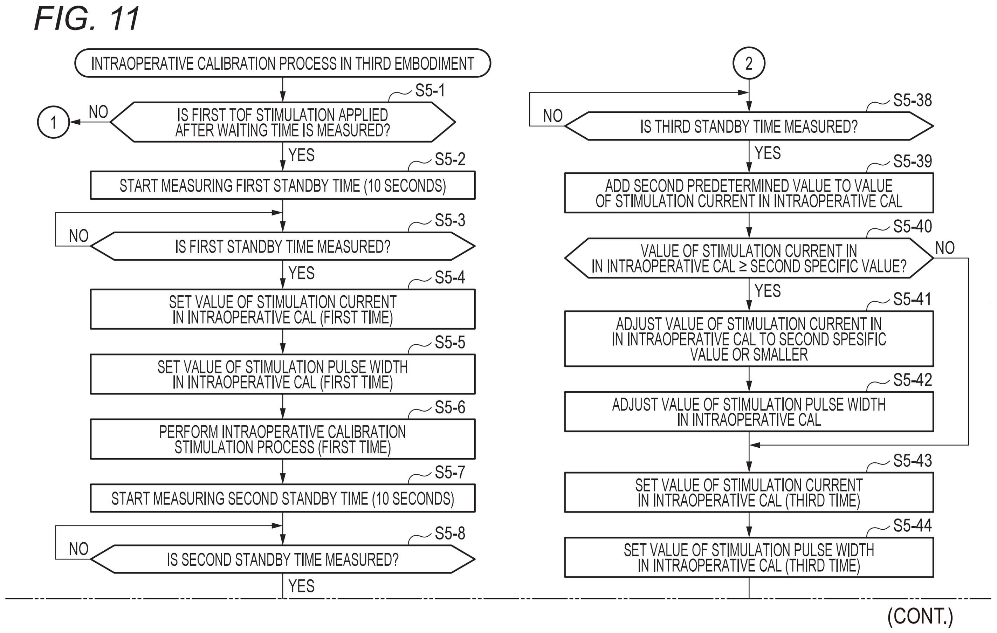

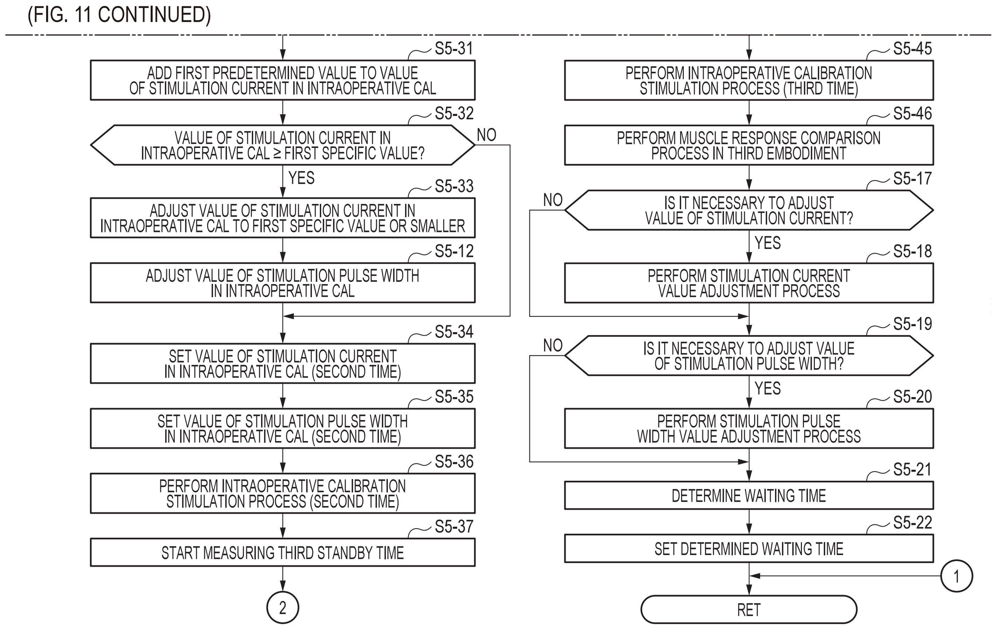

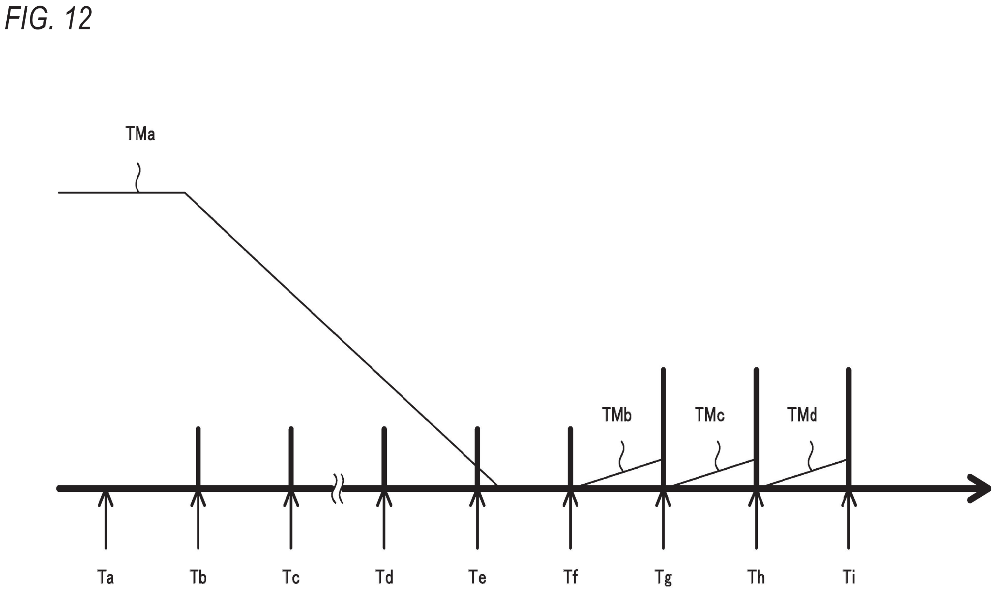

Hereinafter, a third embodiment of the presently disclosed subject matter will be described with reference to FIGS. 11 and 12. In the third embodiment, description of points common to the first embodiment and the second embodiment will be omitted, and only points different from the first embodiment and the second embodiment will be described.

The memory 12 in the third embodiment can include a third standby time timer TMd for measuring a third standby time in addition to the waiting time timer TMa, the first standby time timer TMb, and the second standby time timer TMc. The controller 10 that performs time measurement using the waiting time timer TMa, the first standby time timer TMb, the second standby time timer TMc, and the third standby time timer TMd constitutes a "time measurer" in the presently disclosed subject matter.

### Subroutine of Intraoperative Calibration Process in Third Embodiment

A subroutine of the intraoperative calibration process in the third embodiment will be described with reference to FIG. 11.

In step S5-31, the controller 10 performs a process of adding a first predetermined value to the value of the stimulation current. Specifically, after the measurement of the second standby time is completed, the controller 10 performs a process of adding α mA to the value of the stimulation current in the intraoperative calibration stimulation process (first time). Here, in the present embodiment, the first predetermined value is α mA, but the value of the first predetermined value can be appropriately set. When the process of step S5-31 ends, the process proceeds to step S5-32.

In step S5-32, the controller 10 performs a process of determining whether the value of the stimulation current is a value equal to or larger than a first specific value. Specifically, as a result of performing the process of step S5-31, the controller 10 performs the process of determining whether the value is the value equal to or larger than the first specific value, which is the ceiling. Here, in the present embodiment, the first specific value is β mA, but the value of the first specific value can be appropriately set. When it is determined that the value of the stimulation current is the value equal to or larger than the first specific value (step S5-32 = YES), the process proceeds to step S5-33. On the other hand, when it is determined that the value of the stimulation current is a value not equal to or larger than the first specific value (step S5-32 = NO), the process proceeds to step S5-34.

In step S5-33, the controller 10 performs a process of adjusting the value of the stimulation current to a value equal to or smaller than the first specific value. When the process of step S5-33 ends, the process proceeds to step S5-12.

In step S5-34, the controller 10 performs the process of setting the value of the stimulation current in the intraoperative calibration (second time). Specifically, when the controller 10 determines that the value of the stimulation current in the intraoperative calibration is equal to or larger than the first specific value (step S5-32 = YES), the value adjusted by the process of step S5-33 is set as the value of the stimulation current in the intraoperative calibration (second time). On the other hand, when it is determined that the value of the stimulation current in the intraoperative calibration is not equal to or larger than the first specific value (step S5-32 = NO), a value obtained by adding the first predetermined value to the value of the stimulation current in the intraoperative calibration (first time) is set as the value of the stimulation current in the intraoperative calibration (second time). When the process of step S5-34 ends, the process proceeds to step S5-35.

In step S5-35, the controller 10 performs the process of setting the value of the stimulation pulse width in the intraoperative calibration (second time). Specifically, when it is determined that the value of the stimulation current in the intraoperative calibration is equal to or larger than the first specific value (step S5-32 = YES), the controller 10 performs the process of setting the value of the stimulation pulse width adjusted by the process of step S5-12. When it is determined that the value of the stimulation current in the intraoperative calibration is not equal to or larger than the first specific value (step S5-32 = NO), the process of setting the stimulation pulse width set by the process of step S5-5 is performed. When the process of step S5-35 ends, the process proceeds to step S5-36.

In step S5-36, the controller 10 performs the intraoperative calibration stimulation process (second time). Specifically, the controller 10 performs a process of applying a stimulation at a stimulation intensity having the value of the stimulation current set by the process of step S5-34 and the value of the stimulation pulse width set by the process of step S5-35. When the process of step S5-36 ends, the process proceeds to step S5-37.

In step S5-37, the controller 10 performs a process of starting measuring the third standby time. Specifically, measurement of the third standby time is started by counting up the value of the third standby time timer TMd. Here, in the present embodiment, the third standby time is set to 10 seconds, but other values can be appropriately set. When the process of step S5-37 ends, the process proceeds to step S5-38.

In step S5-38, the controller 10 performs a process of determining whether the third standby time is measured. Specifically, the controller 10 performs a process of determining whether the value of the third standby time timer TMd is 10 seconds, thereby performing the process of determining whether the third standby time is measured. When it is determined that the third standby time is measured (step S5-38 = YES), the process proceeds to step S5-39. On the other hand, when it is determined that the third standby time is not measured (step S5-38 = NO), the process of step S5-38 is repeatedly performed until the third standby time is measured.

In step S5-39, the controller 10 performs a process of adding a second predetermined value to the value of the stimulation current. Specifically, after the measurement of the third standby time is completed, the controller 10 performs a process of adding γ mA to the value of the stimulation current in the intraoperative calibration stimulation process (second time). Here, in the present embodiment, the second predetermined value is γ mA, but the value of the second predetermined value can be appropriately set. When the process of step S5-39 ends, the process proceeds to step S5-40.

In step S5-40, the controller 10 performs a process of determining whether the value of the stimulation current is a value equal to or larger than the second specific value. Specifically, as a result of performing the process of step S5-39, the controller 10 performs the process of determining whether the value is the value equal to or larger than the second specific value, which is the ceiling. Here, in the present embodiment, the second specific value is δ mA, but the value of the second specific value can be appropriately set. When it is determined that the value of the stimulation current is the value equal to or larger than the second specific value (step S5-40 = YES), the process proceeds to step S5-41. On the other hand, when it is determined that the value of the stimulation current is a value not equal to or larger than the second specific value (step S5-40 = NO), the process proceeds to step S5-43.

In step S5-41, the controller 10 performs a process of adjusting the value of the stimulation current to the value equal to or smaller than the second specific value. When the process of step S5-41 ends, the process proceeds to step S5-42.

In step S5-42, the controller 10 performs the process of adjusting the value of the stimulation pulse width in the intraoperative calibration. Since this process is a process which is the same as the process of step S5-12, the description thereof will be omitted. When the process of step S5-42 ends, the process proceeds to step S5-43.

In step S5-43, the controller 10 performs a process of setting a value of the stimulation current in the intraoperative calibration (third time). Specifically, when the controller 10 determines that the value of the stimulation current in the intraoperative calibration is equal to or larger than the second specific value (step S5-40 = YES), the value adjusted by the process of step S5-41 is set as the value of the stimulation current in the intraoperative calibration (third time). On the other hand, when it is determined that the value of the stimulation current in the intraoperative calibration is not equal to or larger than the second specific value (step S5-40 = NO), a value obtained by adding the second predetermined value to the value of the stimulation current in the intraoperative calibration (second time) is set as the value of the stimulation current in the intraoperative calibration (third time). When the process of step S5-42 ends, the process proceeds to step S5-43.

In step S5-44, the controller 10 performs a process of setting a value of the stimulation pulse width in the intraoperative calibration (third time). Specifically, when it is determined that the value of the stimulation current in the intraoperative calibration is equal to or larger than the second specific value (step S5-40 = YES), the controller 10 performs the process of setting the value of the stimulation pulse width adjusted by the process of step S5-42. When it is determined that the value of the stimulation current in the intraoperative calibration is not equal to or larger than the second specific value (step S5-40 = NO), the process of setting the stimulation pulse width set by the process of step S5-35 is performed. When the process of step S5-44 ends, the process proceeds to step S5-45.

In step S5-45, the controller 10 performs an intraoperative calibration stimulation process (third time). Specifically, the controller 10 performs a process of applying a stimulation at a stimulation intensity having the value of the stimulation current set by the process of step S5-43 and the value of the stimulation pulse width set by the process of step S5-44. When the process of step S5-45 ends, the process proceeds to step S5-46.

In step S5-46, the controller 10 performs the muscle response comparison process in the third embodiment. Specifically, the controller 10 performs a process of comparing the muscle response in the intraoperative calibration stimulation process (first time) in step S5-6, the muscle response in the intraoperative calibration stimulation process in step S5-36, and the muscle response in the intraoperative calibration stimulation process in step S5-45. When the process of step S5-46 ends, the process proceeds to step S5-17.

### Timing Chart of Intraoperative Calibration in Third Embodiment

Next, a timing chart of the intraoperative calibration in the third embodiment will be described with reference to FIG. 12.

The timing Ta in FIG. 12 is the timing at which the preoperative calibration is performed. The timing Tb in FIG. 12 is the timing at which the first TOF stimulation is performed after the power source of the muscle relaxation monitoring device 1 is turned on. The timing Tc in FIG. 12 is the timing at which the TOF stimulation of the second time is performed after the power source of the muscle relaxation monitoring device 1 is turned on. The timing Td in FIG. 12 is the timing at which the TOF stimulation of the N-th time is performed after the power source of the muscle relaxation monitoring device 1 is turned on. The timing Te in FIG. 12 is the timing at which the TOF stimulation is performed immediately before the measurement of the waiting time is completed.

The timing Tf in FIG. 12 is the timing at which the first TOF stimulation is performed immediately after the value of the waiting time timer TMa becomes 0 and the measurement of the waiting time is completed. At this time, the measurement of the first standby time is started. The timing Tg in FIG. 12 is a timing at which the first standby time is measured and the intraoperative calibration stimulation process (first time) is performed. At this time, the measurement of the second standby time is started.

The timing Th in FIG. 12 is a timing at which the second standby time is measured and the intraoperative calibration stimulation process (second time) is performed. At this time, in the third embodiment, the measurement of the third standby time is started. Subsequently, a timing Ti in FIG. 12 is a timing at which the value of the third standby time timer TMd becomes 10 seconds and the measurement of the third standby time is completed. At this time, in the third embodiment, the intraoperative calibration stimulation process (third time) is performed.

Here, in the muscle response comparison process in the third embodiment, the process of comparing the muscle response in the intraoperative calibration stimulation process (first time), the muscle response in the intraoperative calibration stimulation process (second time), and the muscle response in the intraoperative calibration stimulation process (third time) is performed. This makes it possible to perform a fine control in the intraoperative calibration process as compared with the intraoperative calibration process in the first embodiment and the intraoperative calibration process in the second embodiment.

In the muscle response comparison process in the third embodiment, three muscle responses of the muscle response in the intraoperative calibration stimulation process (first time), the muscle response in the intraoperative calibration stimulation process (second time), and the muscle response in the intraoperative calibration stimulation process (third time) are compared, but four or more muscle responses may be compared. This makes it possible to perform a finer control in the intraoperative calibration process.

### Other Embodiments

Hereinafter, other embodiments will be described.

In the embodiments described above, the measurement of the waiting time is started when the first TOF stimulation is performed, but the presently disclosed subject matter is not limited thereto. For example, the measurement of the waiting time may be started when a predetermined TOF stimulation of the second time or subsequent times is performed, or the measurement of the waiting time may be started when the communication unit 13 inputs a predetermined signal (for example, a signal having information indicating that the patient has changed) from the monitor communication unit 21. The measurement of the waiting time may be started when the preoperative calibration is performed.

In the embodiments described above, the measurement of the first standby time is started based on the timing at which the first TOF stimulation is performed after the measurement of the waiting time is completed, but the presently disclosed subject matter is not limited thereto. For example, the measurement of the first standby time may be started based on the timing at which the TOF stimulation is performed immediately before the measurement of the waiting time is completed, or the measurement of the first standby time may be started based on the timing at which the measurement of the waiting time is completed.

In the embodiments described above, the first standby time is the same time (10 seconds) as the second standby time, but the first standby time may be a time different from the second standby time. Specifically, the first standby time may be shorter than the second standby time, or may be longer than the second standby time.

In the embodiments described above, the first standby time is the same time (10 seconds) as the third standby time, but the first standby time may be a time different from the third standby time. Specifically, the first standby time may be shorter than the third standby time, or may be longer than the third standby time.

In the embodiments described above, the second standby time is the same time (10 seconds) as the third standby time, but the second standby time may be a time different from the third standby time. Specifically, the second standby time may be shorter than the third standby time, or may be longer than the third standby time. However, it is desirable that each of the first standby time, the second standby time, and the third standby time is a time which is not affected by fading and allows a balanced level of the fine control.

In the embodiments described above, when it is determined that the value of the stimulation current in the intraoperative calibration is the value equal to or larger than the specific value, the value of the stimulation current in the intraoperative calibration is adjusted to the value equal to or smaller than the specific value, but the value of the stimulation current in the intraoperative calibration may be adjusted to a value smaller than the specific value. The same applies to a case where the value of the stimulation current in the intraoperative calibration is the value equal to or larger than the first specific value or a case where the value of the stimulation current in the intraoperative calibration is the value equal to or larger than the second specific value.

When it is determined that the value of the stimulation current in the intraoperative calibration is the value larger than the specific value, the value of the stimulation current in the intraoperative calibration may be adjusted to the value equal to or smaller than the specific value. Same or similarly, when it is determined that the value of the stimulation current in the intraoperative calibration is the value larger than the first specific value or it is determined that the value of the stimulation current in the intraoperative calibration is the value larger than the second specific value, the value of the stimulation current in the intraoperative calibration may be adjusted to the value equal to or smaller than the first specific value or the value equal to or smaller than the second specific value.

In the embodiments described above, the waiting time is measured by setting 10 minutes or 30 minutes in the waiting time timer TMa provided in the memory 12 and counting down, but the waiting time may be measured by counting up the value of the waiting time timer TMa.

In the embodiments described above, the first standby time is measured by counting up the value of the first standby time timer TMb, but the first standby time timer TMb may be set to 10 seconds and counted down. Same or similarly, the second standby time and the third standby time are measured by counting up the value of the second standby time timer TMc and the value of the third standby time timer TMd, but the second standby time timer TMc and the third standby time timer TMd may be set 10 seconds and counted down.

In the embodiments described above, the threshold value A is a value determined in advance, but may be, for example, a value that can be set and changed when a user of the muscle relaxation monitoring device 1 operates the operation unit 11.

In the embodiments described above, the intraoperative calibration stimulation process (first time) is performed when the measurement of the first standby time is completed, but the intraoperative calibration stimulation process may be performed at any timing by the user of the muscle relaxation monitoring device 1. For example, the intraoperative calibration stimulation process (first time) may be performed when the user of the muscle relaxation monitoring device 1 operates the operation unit 11, or the intraoperative calibration stimulation process (first time) may be performed when a specific signal is input from the monitor communication unit 21. The same applies to the intraoperative calibration stimulation process (second time) and the intraoperative calibration stimulation process (third time).

In the embodiments described above, the value of the stimulation current in the intraoperative calibration stimulation process (second time) is adjusted by adding the predetermined value to the value of the stimulation current in the intraoperative calibration stimulation process (first time) in principle, but may be adjusted by adding the predetermined value to the value of the stimulation current in the intraoperative calibration stimulation process (first time). In this case, in principle, the value of the stimulation current in the intraoperative calibration stimulation process (first time) is a value larger than the value of the stimulation current in the intraoperative calibration stimulation process (second time) to which the predetermined value is not added.

In the embodiments described above, when the stimulation intensity is adjusted, while adding 100 µsec to the value of the stimulation pulse width does not involve the adjustment of simply subtracting a constant value from the value of the stimulation current, but when 100 µsec is added to the value of the stimulation pulse width, the stimulation intensity may be adjusted by simply subtracting a constant value from the value of the stimulation current. Accordingly, the program stored in the memory 12 can be simplified, and the process load of the controller 10 can be reduced.

In the embodiments described above, when the TOF ratio is detected as the myoelectric-derived relaxation degree, since there is a constant amplitude, the process of resetting the waiting time timer TMa to the initial value of 30 minutes is performed, but the value reset to the waiting time timer TMa may not be the initial value. For example, the value reset to the waiting time timer TMa may be a value (for example, 35 minutes) larger than the initial value or a value (for example, 25 minutes) smaller than the initial value.

In the embodiments described above, the first predetermined value (α mA) may be a value the same as or a value different from the second predetermined value (γ mA). That is, the first predetermined value (α mA) may be a value larger or a value smaller than the second predetermined value (γ mA). Same or similarly, the first specific value (β mA) may be a value the same as or a value different from the second specific value (δ mA). That is, the first specific value (β mA) may be a value larger or a value smaller than the second specific value (δ mA).

In the embodiments described above, the potential sensing type muscle relaxation monitoring device 1 that applies the stimulation current to the patient and monitors the muscle relaxation degree of the patient based on the potential generated when the muscle of the patient is to be moved has been described as an example, but the presently disclosed subject matter is not limited thereto, and the muscle relaxation monitoring device may be an acceleration sensing type muscle relaxation monitoring device 1 that sequentially observes a muscle contraction state of the muscle as a site to be observed using an acceleration transducer.

As described above, the muscle relaxation monitoring device 1 according to the presently disclosed subject matter can include the stimulator 8 that performs the TOF stimulation on the nerve of the patient and the physiological signal detector 9 that detects the muscle response of the patient. The controller 10 applies the stimulation to the patient via the stimulator 8 by performing the intraoperative calibration stimulation process (first time) and the intraoperative calibration stimulation process (second time). Then, the muscle response when the stimulation is applied by the intraoperative calibration stimulation process (first time) is compared with the muscle response when the stimulation is applied by the intraoperative calibration stimulation process (second time), and the value of the stimulation current and/or the value of the stimulation pulse width in the TOF stimulation performed thereafter is adjusted. Accordingly, it is possible to provide the muscle relaxation monitoring device and the medical device system capable of setting the value of the stimulation current to an optimum value during surgery.

Although the embodiments of the presently disclosed subject matter is described with reference to the drawings, specific configurations are not limited to the embodiments, and changes in design and the like without departing from the gist of the presently disclosed subject matter are also included in the presently disclosed subject matter. The embodiments illustrated in the drawings can be combined with each other as long as there is no contradiction or problem in the purpose, configuration, and the like. In addition, the contents described in each drawing can be independent embodiments, and the embodiments of the presently disclosed subject matter are not limited to one embodiment in which each drawing is combined.

In the muscle relaxation monitoring device according to the presently disclosed subject matter, the stimulator applies the second stimulation based on measurement of a second time by the time measurer after the first stimulation is applied.

In the muscle relaxation monitoring device according to the presently disclosed subject matter, the stimulator applies the second stimulation by adding a predetermined value to the first current value and/or the first pulse width value when the first stimulation is applied.

In the muscle relaxation monitoring device according to the presently disclosed subject matter, the stimulator adjusts the second current value when the second stimulation is applied to a value equal to or smaller than a specific value, when a result of adding the predetermined value to the first current value when the first stimulation is applied is equal to or larger than the specific value.

In the muscle relaxation monitoring device according to the presently disclosed subject matter, the stimulator adjusts the second pulse width value to a value larger than the first pulse width value when a result of adding the predetermined value to the first current value when the first stimulation is applied is equal to or larger than a specific value.

In the muscle relaxation monitoring device according to the presently disclosed subject matter, the stimulator adjusts the predetermined current value to a value equal to or larger than the first current value and adjusts the predetermined pulse width value to a value equal to or larger than the first pulse width value according to a result of comparing the physiological response detected when the first stimulation is applied with the physiological response detected when the second stimulation is applied.

In the muscle relaxation monitoring device according to the presently disclosed subject matter, the stimulator adjusts the predetermined current value to a value equal to or smaller than the second current value and adjusts the predetermined pulse width value to a value equal to or smaller than the second pulse width value according to a result of comparing the physiological response detected when the first stimulation is applied with the physiological response detected when the second stimulation is applied.

In the muscle relaxation monitoring device according to the presently disclosed subject matter, the stimulator adjusts the predetermined current value to a value equal to or smaller than the first current value and adjusts the predetermined pulse width value to a value equal to or larger than the first pulse width value according to a result of comparing the physiological response detected when the first stimulation is applied with the physiological response detected when the second stimulation is applied.

In the muscle relaxation monitoring device according to the presently disclosed subject matter, the stimulator adjusts the predetermined current value to a value equal to or larger than the second current value and adjusts the predetermined pulse width value to a value equal to or smaller than the second pulse width value according to a result of comparing the physiological response detected when the first stimulation is applied with the physiological response detected when the second stimulation is applied.

In the muscle relaxation monitoring device according to the presently disclosed subject matter, the time measurer starts measuring the preparation time based on application of a predetermined stimulation by the stimulator after a power source is turned on.

### REFERENCE SIGNS LIST

1 muscle relaxation monitoring device
2 stimulation electrode
2a first stimulation electrode
2b second stimulation electrode
3 recording electrode
3a first recording electrode 3b second recording electrode 4 neutral electrode
5 wiring
5a first wiring
5b second wiring
6 signal line
6a first signal line
6b second signal line
7 ground line
8 stimulator
9 physiological signal detector
10 controller
11 operation unit
12 memory
13 communication unit
20 bedside monitor
21 monitor communication unit
22 monitor controller
23 monitor memory
A threshold value
L upper limb
Sa saturation point
TMa waiting time timer
TMb first standby time timer
TMc second standby time timer
TMd third standby time timer

## Claims

1. A muscle relaxation monitoring device comprising:
a stimulator configured to apply a stimulation having a predetermined current value and a predetermined pulse width value to a nerve of a living body; and
a physiological response detector configured to detect a physiological response, wherein
the stimulator
applies a first stimulation having a first current value and a first pulse width value, and applies a second stimulation having a second current value and a second pulse width value after applying the first stimulation, and
compares the physiological response detected when the first stimulation is applied with the physiological response detected when the second stimulation is applied, and adjusts the predetermined current value and/or the predetermined pulse width value to be applied to the living body.

2. The muscle relaxation monitoring device according to claim 1, further comprising:
a time measurer configured to perform predetermined time measurement, wherein
the time measurer
starts measuring a preparation time based on satisfaction of a predetermined condition, and the stimulator
applies the first stimulation based on measurement of a first time shorter than the preparation time with reference to a stimulation applied immediately before the preparation time is measured or a stimulation applied immediately after the preparation time is measured, when the preparation time is measured by the time measurer.

3. The muscle relaxation monitoring device according to claim 2, wherein
the stimulator
applies the second stimulation based on measurement of a second time by the time measurer after the first stimulation is applied.

4. The muscle relaxation monitoring device according to claim 1, wherein
the stimulator
applies the second stimulation by adding a predetermined value to the first current value and/or the first pulse width value when the first stimulation is applied.

5. The muscle relaxation monitoring device according to claim 4, wherein
the stimulator
adjusts the second current value when the second stimulation is applied to a value equal to or smaller than a specific value, when a result of adding the predetermined value to the first current value when the first stimulation is applied is equal to or larger than the specific value.

6. The muscle relaxation monitoring device according to claim 4 or claim 5, wherein
the stimulator
adjusts the second pulse width value to a value larger than the first pulse width value when a result of adding the predetermined value to the first current value when the first stimulation is applied is equal to or larger than a specific value.

7. The muscle relaxation monitoring device according to claim 1, wherein
the stimulator
adjusts the predetermined current value to a value equal to or larger than the first current value and adjusts the predetermined pulse width value to a value equal to or larger than the first pulse width value according to a result of comparing the physiological response detected when the first stimulation is applied with the physiological response detected when the second stimulation is applied.

8. The muscle relaxation monitoring device according to claim 1 or claim 7, wherein
the stimulator
adjusts the predetermined current value to a value equal to or smaller than the second current value and adjusts the predetermined pulse width value to a value equal to or smaller than the second pulse width value according to a result of comparing the physiological response detected when the first stimulation is applied with the physiological response detected when the second stimulation is applied.

9. The muscle relaxation monitoring device according to claim 1, wherein
the stimulator
adjusts the predetermined current value to a value equal to or smaller than the first current value and adjusts the predetermined pulse width value to a value equal to or larger than the first pulse width value according to a result of comparing the physiological response detected when the first stimulation is applied with the physiological response detected when the second stimulation is applied.

10. The muscle relaxation monitoring device according to claim 1, wherein
the stimulator
adjusts the predetermined current value to a value equal to or larger than the second current value and adjusts the predetermined pulse width value to a value equal to or smaller than the second pulse width value according to a result of comparing the physiological response detected when the first stimulation is applied with the physiological response detected when the second stimulation is applied.

11. The muscle relaxation monitoring device according to claim 2, wherein
the time measurer
starts measuring the preparation time based on application of a predetermined stimulation by the stimulator after a power source is turned on.

12. A medical device system comprising:
a muscle relaxation monitoring device configured to monitor a muscle relaxation degree of a patient; and
a medical device configured to communicate with the muscle relaxation monitoring device, wherein
the muscle relaxation monitoring device includes
a stimulator configured to apply a stimulation having a predetermined current value and a predetermined pulse width value to a nerve of a living body,
a physiological response detector configured to detect a physiological response, and
a communication unit configured to output information to the medical device,
the stimulator
applies a first stimulation having a first current value and a first pulse width value, and applies a second stimulation having a second current value and a second pulse width value after applying the first stimulation, and
compares the physiological response detected when the first stimulation is applied with the physiological response detected when the second stimulation is applied, and adjusts the predetermined current value and/or the predetermined pulse width value to be applied to the living body,
the communication unit
outputs comparison information on a result of the comparison to the medical device, and
the medical device
includes a medical device controller that performs a control according to the comparison information output from the communication unit.
